# EUROPEAN PATENT APPLICATION

(11) **EP 4 095 158 A1**
(43) Date of publication of application: **30.11.2022**
(21) Application number: 21744312.6
(22) Date of filing: 19.01.2021
(51) Int. Cl.: C07K 16/28, A61K 39/395, A61P 37/06

(54) **PHARMACEUTICAL COMPOSITION CONTAINING ANTI-BTLA ANTIBODY AND USE THEREOF**

(30) Priority: 20.01.2020 CN 202010066932
(71) Applicant: Shanghai Junshi Biosciences Co., Ltd., Pilot Free Trade Zone Shanghai 201210 (CN); Suzhou Junmeng Biosciences Co., Ltd., Jiangsu 215002 (CN)
(72) Inventor: LIU, Hongchuan, Suzhou, Jiangsu 215002 (CN); LIU, Peixiang, Suzhou, Jiangsu 215002 (CN); ZHANG, Jing, Suzhou, Jiangsu 215002 (CN); ZHOU, Yuehua, Suzhou, Jiangsu 215002 (CN); LIU, Hui, Suzhou, Jiangsu 215002 (CN); CHEN, Xueru, Suzhou, Jiangsu 215002 (CN); WANG, Jing, Suzhou, Jiangsu 215002 (CN); FENG, Hui, Suzhou, Jiangsu 215002 (CN); YAO, Sheng, Suzhou, Jiangsu 215002 (CN)
(74) Representative: V.O.
(86) International application number: PCT/CN2021/072660
(87) International publication number: WO 2021/147846

(57) **Abstract**

The present invention provides a stable pharmaceutical composition comprising an anti-BTLA (B and T lymphocyte attenuator) antibody and use thereof in medicines. The pharmaceutical composition comprises an anti-BTLA antibody and a buffer, further comprises at least one stabilizer, and optionally further comprises a surfactant.

## Description

### TECHNICAL FIELD

The present invention relates to the field of therapeutic pharmaceutical composition, and particularly, to the field of pharmaceutical formulation, wherein the pharmaceutical composition comprises a humanized antibody that specifically binds to B and T lymphocyte attenuator (BTLA).

### BACKGROUND

Positive and negative co-stimulatory signals play a decisive role in regulating the activity of B cells and T cells, and molecules that mediate these signals have been shown to be effective targets for immune modulators. In addition to the involvement of T cell receptor (TCR), positive co-stimulation is required for optimal activation of naive T cells, whereas negative co-stimulation is considered to be required for the acquisition of autoimmune tolerance and the termination of effector T cell function. Upon interacting with B7.1 or B7.2 on the surface of antigen-presenting cells (APCs), the prototype T-cell co-stimulatory molecule CD28 signals the promotion of T-cell proliferation and differentiation in response to the involvement of TCR, while CD28 homolog cytotoxic T-lymphocyte antigen-4 (CTLA-4) mediates the inhibition of T-cell proliferation and effector function (Chambers et al., Ann. Rev. Immunol., 19: 565-594, 2001; Egen et al., Nature Immunol., 3: 611-618, 2002). Several new molecules homologous to the B7 family have been found (Abbas et al., Nat.Med., 5: 1345-6, 1999; Coyle et al., Nat. Immunol., 2: 203-9, 2001; Carreno et al., Annu. Rev. Immunol., 20: 29-53, 2002; Liang et al., Curr. Opin. Immunol., 14: 384-90, 2002) and the elucidation for their roles in T cell activation just begins.

B and T lymphocyte attenuator (BTLA) is a member of the CD28 family, which also includes CD28, ICOS, CTLA-4 and PD-1. Based on the functional effect of increasing T cell proliferation by the addition of monoclonal antibody, the first members of this family, CD28 and ICOS, are found to have an immune activation effect (Hutloff et al., 1999), and BTLA, CTLA-4, PD-1 and the like are described as negative regulatory proteins. Several *in vivo* studies have demonstrated the inhibitory effect of BTLA on lymphocyte responses. BTLA-deficient mice acquired by Murphy and his colleagues (Washington University St.Louis) show a 3-fold increase in IgG production in response to T-dependent antigens. In addition, T cells and B cells isolated from BTLA⁻mice show stronger proliferative responses to antigen-receptor stimulation with CD3- and anti-IgM (Watanabe, 2003). In an overexpression study, BTLA is found to associate with B cell receptor complexes as well as T cell receptors. In line with the results of this study, in BTLA-deficient lymphocytes, antigen-receptor independent stimulation using ConA (T cells) or LPS (B cells) is not affected and cannot be regulated even when using an anti-BTLA antibody. BTLA-knockout mice have been shown to develop spontaneous autoimmune diseases over time and to have a shortened lifespan (Oya, 2008). The disease severity increases for BTLA-knockout mice with experimental autoimmune encephalomyelitis (EAE) and allergic airway inflammation models, both of which rely on T cell activity (Watanabe, 2005; Deppong, 2006).

Herpesvirus entry mediator (HVEM) has been shown to be a ligand of BTLA (Scully et al, 2005). HVEM is a type I transmembrane glycoprotein with 4 extracellular cysteine-rich domains (CRDs) containing 6 pseudorepetitive cysteines, and it is a member of the TNF receptor superfamily. BTLA and HVEM regulate the function of T cells and APC mainly through dynamic expression on the cell surface. The binding of BTLA to ligand not only inhibits T cell proliferation and down-regulates the T cell activation marker CD25, but also inhibits the production of IFN-γ, IL-2, IL-4, IL-10 and the like, but does not induce cell apoptosis. The binding of HVEM to BTLA results in the down-regulation of T cell activation and proliferation (Sedy, 2005). These findings indicate that BTLA expression or BTLA-HVEM binding is closely related to T cell activation and proliferation.

An antibody can be used as a therapeutic agent. Some antibodies may cause unwanted immunogenicity of the antibody when used as therapeutic agents *in vivo.* Repeated use of a monoclonal antibody in humans results in an immune response against the therapeutic antibody (e.g., human anti-mouse antibody or HAMA), due to the rodent origin of the most monoclonal antibodies. Such immune responses result in at least a loss of therapeutic efficacy and, at most, a potentially lethal allergic reaction. One method for reducing the immunogenicity of rodent antibodies includes producing chimeric antibodies, in which the mouse variable region (Fv) is fused to the human constant region (Liu et al. (1987) Proc.Natl.Acad.Sci. USA 84: 3439-43). However, mice injected with a hybrid of the human variable region and the mouse constant region develop a strong antibody response against the human variable region, which indicates that the retention of the intact rodent Fv region in such chimeric antibodies may still cause deleterious immunogenicity in patients.

In addition, grafting of the complementarity determining region (CDR) loops of the rodent variable domain onto the human framework (i.e., humanization) has been used to further minimize rodent sequences. Jones et al. (1986) Nature 321: 522; Verhoeyen et al. (1988) Science 239: 1534. However, CDR loop exchange still fails to uniformly produce an antibody with the same binding properties as the initial antibody. In humanized antibodies, changes in framework residues (FRs) (residues involved in CDR loop support) are often required to maintain the antigen-binding affinity. Kabat et al. (1991) J. Immunol. 147: 1709. Although it has been reported the use of CDR grafting and framework residue retention in many humanized antibody constructs, it is difficult to predict whether a particular sequence will produce an antibody with the desired binding properties and, occasionally, biological properties. See, e.g., Queen et al., (1989) Proc. Natl. Acad. Sci. USA 86: 10029; Gorman et al., (1991) Proc. Natl. Acad. Sci. USA 88: 4181; and Hodgson, (1991) Biotechnology (NY), 9: 421-5. Furthermore, in most of the existing studies, different human sequences are adopted for the light and heavy chain variable sequences of animals, rendering the predictability of such studies questionable. The sequences of known antibodies, or more generally antibodies having a known X-ray crystal structure such as antibodies NEW and KOL, have been used. See, e.g., Jones et al., *supra;* Verhoeyen et al., *supra;* and Gorman et al., *supra.* Exact sequence information has been reported for a few humanized constructs.

There is a need for anti-BTLA antibodies, particularly anti-BTLA monoclonal antibodies, for use in the treatment of human disorders (e.g., inflammatory disorders, autoimmune disorders and proliferative disorders). Such antibodies may preferably have low immunogenicity in human subjects, allowing repeated administration without adverse immune responses.

Thus, there is a need in the art for protein formulations with high stability.

### SUMMARY

The pharmaceutical composition provided by the present invention is a highly stable pharmaceutical composition containing a humanized antibody specifically binding to BTLA. In particular, the present inventors have found that the combination of trehalose with sodium chloride may significantly improve the stability of the pharmaceutical composition.

The present invention provides a pharmaceutical composition comprising: (1) a buffer; and (2) an anti-BTLA antibody or an antigen-binding fragment thereof.

In some embodiments, the anti-BTLA antibody or the antigen-binding fragment thereof has an HCDR1, an HCDR2 and an HCDR3 having amino acid sequences set forth in SEQ ID NO: 1, SEQ ID NO: 2 and SEQ ID NO: 3, respectively, and an LCDR1, an LCDR2 and an LCDR3 having amino acid sequences set forth in SEQ ID NO: 4, SEQ ID NO: 5 and SEQ ID NO: 6, respectively.

In some embodiments, the anti-BTLA antibody or the antigen-binding fragment thereof in the pharmaceutical composition has a concentration of about 1-200 mg/mL, preferably about 5-100 mg/mL, and preferably about 10-50 mg/mL; more preferably, the anti-BTLA antibody or the antigen-binding fragment thereof has a concentration of about 10 mg/mL, 15 mg/mL, 20 mg/mL, 25 mg/mL, 30 mg/mL, 35 mg/mL, 40 mg/mL, 45 mg/mL or 50 mg/mL, preferably about 10 mg/mL, 20 mg/mL or 40 mg/mL.

In some embodiments, the buffer is selected from one or more of an acetic acid buffer, a citric acid buffer and a histidine buffer.

In some embodiments, the buffer is a histidine buffer; preferably, the histidine buffer is selected from a histidine-hydrochloride buffer or a histidine-acetate buffer, preferably a histidine-hydrochloride buffer.

In some embodiments, the histidine-hydrochloride buffer consists of histidine and histidine hydrochloride, preferably L-histidine and L-histidine monohydrochloride. In some embodiments, the histidine buffer is prepared by 1-20 mM L-histidine and 1-20 mM L-histidine monohydrochloride. In some embodiments, the histidine buffer consists of histidine and histidine hydrochloride in a molar ratio of 1:1-1:4. In some embodiments, the histidine buffer consists of histidine and histidine hydrochloride in a molar ratio of 1:1. In some embodiments, the histidine buffer consists of histidine and histidine hydrochloride in a molar ratio of 1:3. In some embodiments, the histidine formulation is a histidine buffer at pH 5.5 prepared by 4.5 mM L-histidine and 15.5 mM L-histidine monohydrochloride. In some embodiments, the histidine formulation is a histidine buffer at pH 6.0 prepared by 15 mM L-histidine and 15 mM L-histidine monohydrochloride.

In some embodiments, the histidine buffer is a histidine-acetic acid buffer, preferably in a molar ratio of 1:1 to 1.5:1; preferably such a buffer has a pH of 5.5 ± 0.3, preferably about 5.5; preferably such a buffer contains 15-20 mM histidine and 12-15 mM acetic acid.

In some embodiments, the buffer is an acetic acid buffer; preferably, the acetic acid buffer is an acetic acid-sodium acetate buffer or an acetic acid-potassium acetate buffer, preferably an acetic acid-sodium acetate buffer.

In some embodiments, the buffer is a citric acid buffer; preferably, the citric acid buffer is a citric acid-sodium citrate buffer.

In some embodiments, the buffer has a concentration of about 1-100 mM, preferably about 5-50 mM, preferably about 10-30 mM, preferably about 20-30 mM, and preferably about 10-20 mM; and non-limiting example of the concentration of the buffer is about 5 mM, 10 mM, 15 mM, 20 mM, 25 mM, 30 mM, 40 mM, 45 mM, 50 mM, 55 mM, 60 mM, 65 mM, 70 mM, 75 mM, 80 mM, 85 mM, 90 mM, 95 mM or 100 mM or in a range with any two of the values as endpoints, preferably about 10 mM, 20 mM or 30 mM.

In some embodiments, the buffer has a pH of about 5.0-6.5, preferably about 5.0-6.0, preferably about 5.5-6.5, preferably about 5.0-5.5, preferably about 5.5-6.0, and preferably about 6.0-6.5, and non-limiting examples of the pH of the buffer are about 5.0, 5.1, 5.2, 5.3, 5.4, 5.5, 5.6, 5.7, 5.8, 5.9, 6.0, 6.1, 6.2, 6.3, 6.4 and 6.5, preferably about 5.0, 5.5 or 6.0.

In some embodiments, the pharmaceutical composition further comprises a stabilizer selected from one or more of sodium chloride, mannitol, sorbitol, sucrose, maltose, xylitol and trehalose. Preferably, the stabilizer is a combination of trehalose and sodium chloride.

In some embodiments, the stabilizer has a concentration of about 20 mM to 300 mM, preferably 50 mM to 300 mM, and more preferably 120 mM to 250 mM.

In some embodiments, the stabilizer is sodium chloride at a concentration of about 50-200 mM; or the stabilizer is mannitol at a concentration of about 100-300 mM; or the stabilizer is sorbitol at a concentration of about 100-300 mM; or the stabilizer is sucrose at a concentration of about 100-300 mM; or the stabilizer is trehalose at a concentration of about 100-300 mM; or the stabilizer is a combination of about 30-100 mM sodium chloride and about 50-200 mM mannitol; the stabilizer is a combination of about 30-100 mM sodium chloride and about 50-200 mM sorbitol; the stabilizer is a combination of about 30-100 mM sodium chloride and about 50-200 mM sucrose; the stabilizer is a combination of about 30-100 mM sodium chloride and about 50-200 mM trehalose.

In some embodiments, the stabilizer is sodium chloride. In some embodiments, the stabilizer is sodium chloride at a concentration of about 50-200 mM, preferably about 100-190 mM, preferably about 120-180 mM, preferably about 130-170 mM, and preferably about 130-150 mM, and non-limiting examples of the concentration of sodium chloride are about 100 mM, 110 mM, 120 mM, 125 mM, 130 mM, 135 mM, 140 mM, 145 mM, 150 mM, 155 mM, 160 mM, 170 mM, 180 mM, 190 mM and 200 mM, preferably 135 mM or 140 mM.

In some embodiments, the stabilizer is mannitol. In some embodiments, the stabilizer is mannitol at a concentration of about 100-300 mM, preferably about 150-300 mM, and preferably about 200-280 mM, and non-limiting examples of the concentration of mannitol are about 200 mM, 210 mM, 220 mM, 230 mM, 240 mM, 250 mM, 260 mM, 270 mM and 280 mM, preferably 240 mM.

In some embodiments, the stabilizer is sorbitol. In some embodiments, the stabilizer is sorbitol at a concentration of about 100-300 mM, preferably about 150-300 mM, and preferably about 200-280 mM, and non-limiting examples of the concentration of sorbitol are about 200 mM, 210 mM, 220 mM, 230 mM, 240 mM, 250 mM, 260 mM, 270 mM and 280 mM, preferably 240 mM.

In some embodiments, the stabilizer is sucrose. In some embodiments, the stabilizer is sucrose at a concentration of about 100-300 mM, preferably about 150-300 mM, and preferably about 200-280 mM, and non-limiting examples of the concentration of sucrose are about 200 mM, 210 mM, 220 mM, 230 mM, 220 mM, 250 mM, 260 mM, 270 mM and 280 mM, preferably 220 mM.

In some embodiments, the stabilizer is trehalose. In some embodiments, the stabilizer is trehalose at a concentration of about 100-300 mM, preferably about 150-300 mM, and preferably about 200-280 mM, and non-limiting examples of the concentration of trehalose are about 180 mM, 200 mM, 210 mM, 220 mM, 230 mM, 240 mM, 250 mM, 260 mM, 270 mM and 280 mM, preferably 220 mM.

In some embodiments, the stabilizer is a combination of sodium chloride and mannitol. In some embodiments, the stabilizer is a combination of about 30-100 mM sodium chloride and about 50-200 mM mannitol, preferably a combination of about 40-80 mM sodium chloride and about 100-180 mM mannitol, and preferably a combination of about 40-60 mM sodium chloride and about 120-160 mM mannitol, and non-limiting examples of the stabilizer are a combination of about 54 mM sodium chloride and about 144 mM mannitol, and a combination of about 50 mM sodium chloride and about 140 mM mannitol.

In some embodiments, the stabilizer is a combination of sodium chloride and sorbitol. In some embodiments, the stabilizer is a combination of about 30-100 mM sodium chloride and about 50-200 mM sorbitol, preferably a combination of about 40-80 mM sodium chloride and about 100-180 mM sorbitol, and preferably a combination of about 40-60 mM sodium chloride and about 120-160 mM sorbitol, and non-limiting examples of the stabilizer are a combination of about 54 mM sodium chloride and about 144 mM sorbitol, and a combination of about 40 mM sodium chloride and about 160 mM sorbitol.

In some embodiments, the stabilizer is a combination of sodium chloride and sucrose. In some embodiments, the stabilizer is a combination of about 30-100 mM sodium chloride and about 50-200 mM sucrose, preferably a combination of about 40-80 mM sodium chloride and about 100-180 mM sucrose, and preferably a combination of about 40-60 mM sodium chloride and about 120-160 mM sucrose, and non-limiting examples of the stabilizer are a combination of about 54 mM sodium chloride and about 132 mM sucrose, and a combination of about 50 mM sodium chloride and about 150 mM sucrose.

In some embodiments, the stabilizer is a combination of sodium chloride and trehalose. In some embodiments, the stabilizer is a combination of about 30-100 mM sodium chloride and about 50-200 mM trehalose, preferably a combination of about 40-80 mM sodium chloride and about 100-180 mM trehalose, and preferably a combination of about 40-60 mM sodium chloride and about 120-160 mM trehalose, and non-limiting examples of the stabilizer are a combination of about 54 mM sodium chloride and about 132 mM trehalose, a combination of about 50 mM sodium chloride and about 140 mM trehalose, and a combination of about 60 mM sodium chloride and about 120 mM trehalose, preferably a combination of about 54 mM sodium chloride and about 132 mM trehalose, or a combination of about 50 mM sodium chloride and about 140 mM trehalose.

In some embodiments, the pharmaceutical composition further comprises a surfactant selected from polysorbate 80, polysorbate 20 and poloxamer 188.

In some embodiments, the surfactant is selected from polysorbate 80.

In some embodiments, the surfactant is selected from polysorbate 20.

In some embodiments, the surfactant has a concentration of about 0.001%-0.1%, preferably about 0.01%-0.05%, and preferably about 0.02%-0.04% (w/v), and non-limiting example of the concentration of the surfactant is about 0.02%, 0.03% or 0.04%, preferably 0.02%.

In some embodiments, the anti-BTLA antibody or the antigen-binding fragment thereof is selected from a murine antibody, a chimeric antibody and a humanized antibody, preferably a humanized antibody.

In some embodiments, the anti-BTLA antibody or the antigen-binding fragment thereof has a heavy chain variable region having an amino acid sequence set forth in SEQ ID NO: 7 and a light chain variable region having an amino acid sequence set forth in SEQ ID NO: 8.

In some embodiments, the anti-BTLA antibody or the antigen-binding fragment thereof has a heavy chain variable region having an amino acid sequence set forth in SEQ ID NO: 7 and a light chain variable region having an amino acid sequence set forth in SEQ ID NO: 9.

In some embodiments, the anti-BTLA antibody or the antigen-binding fragment thereof has a heavy chain variable region having an amino acid sequence set forth in SEQ ID NO: 7 and a light chain variable region having an amino acid sequence set forth in SEQ ID NO: 10.

In some embodiments, the anti-BTLA antibody or the antigen-binding fragment thereof has a heavy chain amino acid sequence set forth in SEQ ID NO: 11 and a light chain amino acid sequence set forth in SEQ ID NO: 12.

In some embodiments, the pharmaceutical composition comprises or consists of components shown as in any one of (1) to (8) below; wherein the anti-BTLA antibody or the antigen-binding fragment thereof is described as in any embodiment of the present invention:
(1) (a) about 10 mg/mL to 50 mg/mL of the anti-BTLA antibody or the antigen-binding fragment thereof; (b) about 10-30 mM histidine buffer at a pH of about 5.0-6.0; (c) a combination of about 30 mM to about 100 mM sodium chloride and about 50-200 mM trehalose; and (d) about 0.01%-0.05% of polysorbate 80; or
(2) (a) about 10 mg/mL to 50 mg/mL of the anti-BTLA antibody or the antigen-binding fragment thereof; (b) about 10-30 mM histidine buffer at a pH of about 5.0-6.0; (c) a combination of about 30 mM to about 100 mM sodium chloride and about 50-200 mM mannitol; and (d) about 0.01%-0.05% of polysorbate 80; or
(3) (a) about 10 mg/mL to 50 mg/mL of the anti-BTLA antibody or the antigen-binding fragment thereof; (b) about 10-30 mM histidine buffer at a pH of about 5.0-6.0; (c) a combination of about 30 mM to about 100 mM sodium chloride and about 50-200 mM sucrose; and (d) about 0.01%-0.05% of polysorbate 80; or
(4) (a) about 10 mg/mL to 50 mg/mL of the anti-BTLA antibody or the antigen-binding fragment thereof; (b) about 10-30 mM histidine buffer at a pH of about 5.0-6.0; (c) a combination of about 30 mM to about 100 mM sodium chloride and about 50-200 mM sorbitol; and (d) about 0.01%-0.05% of polysorbate 80; or
(5) (a) about 10 mg/mL to 50 mg/mL of the anti-BTLA antibody or the antigen-binding fragment thereof; (b) about 10-30 mM histidine buffer at a pH of about 5.0-6.0; (c) about 100 mM to about 300 mM trehalose; and (d) about 0.01%-0.05% of polysorbate 80;
(6) (a) about 10 mg/mL to 50 mg/mL of the anti-BTLA antibody or the antigen-binding fragment thereof; (b) about 10-30 mM citric acid buffer at a pH of about 5.5-6.5; (c) about 100 mM to about 300 mM trehalose; and (d) about 0.01%-0.05% of polysorbate 80;
(7) (a) about 10 mg/mL to 50 mg/mL of the anti-BTLA antibody or the antigen-binding fragment thereof; (b) about 10-30 mM citric acid buffer at a pH of about 5.5-6.5; (c) a combination of about 30 mM to about 100 mM sodium chloride and about 50-200 mM mannitol; and (d) about 0.01%-0.05% of polysorbate 80; and
(8) (a) about 10 mg/mL to 50 mg/mL of the anti-BTLA antibody or the antigen-binding fragment thereof; (b) about 10-30 mM acetic acid buffer at a pH of about 5.0-6.0; (c) a combination of about 30 mM to about 100 mM sodium chloride and about 50-200 mM mannitol; and (d) about 0.01%-0.05% of polysorbate 80.
   In some embodiments, the pharmaceutical composition comprises or consists of components as shown in any one of (9) to (13) below:
(9) (a) about 20 mg/mL of the anti-BTLA antibody or the antigen-binding fragment thereof; (b) about 20 mM histidine buffer at a pH of about 6.0; (c) a combination of about 54 mM sodium chloride and about 132 mM trehalose; and (d) about 0.02% of polysorbate 80; or
(10) (a) about 20 mg/mL of the anti-BTLA antibody or the antigen-binding fragment thereof; (b) about 20 mM histidine buffer at a pH of about 5.5; (c) about 220 mM trehalose; and (d) about 0.02% of polysorbate 80; or
(11) (a) about 20 mg/mL of the anti-BTLA antibody or the antigen-binding fragment thereof; (b) about 20 mM histidine buffer at a pH of about 5.5; (c) a combination of about 50 mM sodium chloride and about 140 mM mannitol; and (d) about 0.02% of polysorbate 80;
(12) (a) about 20 mg/mL of the anti-BTLA antibody or the antigen-binding fragment thereof; (b) about 20 mM histidine buffer at a pH of about 5.5; (c) a combination of about 50 mM sodium chloride and about 140 mM trehalose; and (d) about 0.02% of polysorbate 80; and
(13) (a) about 20 mg/mL of the anti-BTLA antibody or the antigen-binding fragment thereof; (b) about 20 mM histidine buffer at a pH of about 5.0-6.0; (c) a combination of about 50 mM sodium chloride and about 140 mM trehalose; and (d) about 0.02% of polysorbate 80.

In some embodiments, the pharmaceutical composition is a liquid formulation or a lyophilized formulation.

In some embodiments, the pharmaceutical composition is a liquid formulation, such as an aqueous solution formulation.

In some embodiments, the liquid formulation or lyophilized formulation is stable at 2-8 °C for at least 3 months, at least 6 months, at least 12 months, at least 18 months or at least 24 months.

In some embodiments, the aqueous solution or lyophilized formulation is stable at 40 °C for at least 7 days, at least 14 days or at least 28 days.

The present invention also provides use of the pharmaceutical composition in the preparation of a medicament for treating or preventing BTLA-mediated diseases.

In some embodiments, the diseases include tumors, infectious diseases, and inflammatory or autoimmune diseases. The tumors include melanoma, breast cancer, renal cancer, prostate cancer, colon cancer, lung cancer, pancreatic cancer, bone cancer, skin cancer, head or neck cancer, uterine cancer, ovarian cancer, rectal cancer, anal cancer, stomach cancer, testicular cancer, small intestine cancer, cervical cancer, vaginal cancer, Hodgkin's disease, non-Hodgkin's lymphoma, esophageal cancer, cancer of the endocrine system, thyroid cancer, carcinoma of adrenal gland, soft tissue cancer, urethral cancer, chronic or acute leukemia, chronic myeloid leukemia, acute lymphoblastic leukemia, chronic lymphocytic leukemia, childhood solid tumor, lymphocytic lymphoma, bladder cancer, kidney or ureteral cancer, renal pelvis cancer, neoplasms of the central nervous system, primary central nervous system lymphoma, tumor angiogenesis, spinal axis tumor, brain stem glioma, pituitary adenoma, Kaposi's sarcoma, epidermoid carcinoma, squamous cell carcinoma, T-cell lymphoma, environmentally induced cancers including those induced by asbestos, and combinations of the cancers. The autoimmune diseases include organ-specific autoimmune diseases and systemic autoimmune diseases; the organ-specific autoimmune diseases include chronic lymphocytic thyroiditis, hyperthyroidism, insulin-dependent diabetes mellitus, myasthenia gravis, ulcerative colitis, pernicious anemia with chronic atrophic gastritis, goodpasture's syndrome, pemphigus vulgaris, pemphigoid, primary biliary cirrhosis, multiple sclerosis, acute idiopathic polyneuritis and the like; and the systemic autoimmune diseases include systemic lupus erythematosus, rheumatoid arthritis, systemic vasculitis, scleroderma, pemphigus, dermatomyositis, mixed connective tissue disease, autoimmune hemolytic anemia, ulcerative colitis and the like.

### BRIEF DESCRIPTION OF THE DRAWINGS

FIG. 1 shows a comparative experiment on the binding of humanized antibody hu17 to BTLAs of different species.
FIG. 2 shows the effect of hu18 on the tumor volume of MC38-hHVEM cell-transplanted B-hBTLA mice.
FIG. 3 shows the effect of test samples on the body weight of MC38-hHVEM cell-transplanted B-hBTLA mice.

### Definitions and Description

In order to facilitate the understanding of the present invention, certain technical and scientific terms are specifically defined below. Unless otherwise specifically defined herein, all other technical and scientific terms used herein have the same meaning as commonly understood by those of ordinary skill in the art to which the present invention belongs. It should be understood that the present invention is not limited to particular methods, agents, compounds, compositions or biological systems, which can, of course, be modified. It should also be understood that the terms used herein are for the purpose of illustrating particular embodiments only, and are not intended to be limiting.

As used in this specification and the appended claims, the singular forms "a", "an" and "the" include plural referents, unless otherwise specified. Thus, for example, reference to "a polypeptide" includes a combination of two or more polypeptides and the like.

The term "pharmaceutical composition" or "formulation" refers to a mixture containing one or more compounds described herein or a pharmaceutically acceptable salt or prodrug thereof, and other components, such as physiologically acceptable carriers and excipients. The pharmaceutical composition is intended to promote the administration to an organism and facilitate the absorption of the active ingredient, thereby exerting bioactivity.

The term "liquid formulation" refers to a formulation in liquid state and is not intended to refer to a resuspended lyophilized formulation. The liquid formulation of the present invention is stable during storage and its stability is independent of lyophilization (or other state change methods, such as spray drying).

The term "aqueous formulation" refers to a liquid formulation using water as the solvent. In some embodiments, the aqueous formulation is a formulation that requires no lyophilization, spray drying and/or freezing to maintain its stability (e.g., chemical and/or physical stability and/or bioactivity).

The term "excipient" refers to an agent that may be added to a formulation to provide a desired property (e.g., consistency and improved stability) and/or to adjust osmotic pressure. Examples of commonly used excipients include, but are not limited to, sugars, polyols, amino acids, surfactants and polymers.

As used herein, when referring to measurable values (e.g., amounts and durations), "about" is intended to encompass variations of ±20% or ±10% based on the particular value, including ±5%, ±1% and ±0.1%, as such variations are suitable for implementing the disclosed methods.

The term "buffer at a pH of about 5.0-6.5" refers to such an agent that, through the action of its acid/base conjugate components, renders a solution containing the agent resistant to pH changes. The buffer used in the formulation of the present invention may have a pH in a range of about 5.0 to about 6.5, or a pH in a range of about 5.5 to about 6.5, or a pH in a range of about 5.0 to about 6.0.

Examples of "buffer" that control the pH within this range herein include acetate (e.g., sodium acetate), succinate (e.g., sodium succinate), gluconic acid, histidine, histidine hydrochloride, methionine, citrate, phosphate, citrate/phosphate, imidazole, acetic acid, acetate, citrate and a combination thereof, and other organic acid buffers.

The "histidine buffer" is a buffer containing histidine ions. Examples of histidine buffer include histidine and a salt of histidine such as histidine hydrochloride, histidine acetate, histidine phosphate and histidine sulfate, for example, a histidine buffer containing histidine and histidine hydrochloride; the histidine buffer of the present invention also includes a histidine buffer containing histidine and acetate (e.g., sodium salt or potassium salt).

The "citrate buffer" is a buffer containing citrate ions. Examples of citrate buffer include citric acid-sodium citrate, citric acid-potassium citrate, citric acid-calcium citrate, citric acid-magnesium citrate and the like. The preferred citrate buffer is a citric acid-sodium citrate buffer.

The "acetate buffer" is a buffer containing acetate ions. Examples of acetate buffer include acetic acid-sodium acetate, acetic acid-potassium acetate, acetic acid-calcium acetate, acetic acid-magnesium acetate and the like. The preferred acetate buffer is an acetic acid-sodium acetate buffer.

The term "stabilizer" refers to a pharmaceutically acceptable excipient that protects the active pharmaceutical ingredient and/or formulation from chemical and/or physical degradation during manufacture, storage and use. The stabilizers include, but are not limited to, sugars, amino acids, salts, polyols and metabolites thereof as defined below, such as sodium chloride, calcium chloride, magnesium chloride, mannitol, sorbitol, sucrose, trehalose, arginine hydrochloride, arginine, glycine, alanine (a-alanine, β-alanine), betaine, leucine, lysine, glutamic acid, aspartic acid, proline, 4-hydroxyproline, sarcosine, γ-aminobutyric acid (GABA), opines (alanopine, octopine, strombine) and trimethylamine N-oxide (TMAO), human serum albumin (hsa), bovine serum albumin (bsa), α-casein, globulin, α-lactalbumin, LDH, lysozyme, myoglobin, ovalbumin and RNAaseA. Some stabilizers, such as sodium chloride, calcium chloride, magnesium chloride, mannitol, sorbitol and sucrose, may also serve to control osmotic pressure. The stabilizer specifically used in the present invention is selected from one, two and more of polyols, salts and sugars. The preferred salts are sodium chloride, the preferred sugars are sucrose and trehalose, and the preferred polyols are sorbitol and mannitol. The preferred stabilizers are sodium chloride, mannitol, sorbitol, sucrose, trehalose, sodium chloride-sorbitol, sodium chloride-mannitol, sodium chloride-sucrose and sodium chloride-trehalose, more preferably sodium chloride-sorbitol, sodium chloride-mannitol, sodium chloride-sucrose and sodium chloride-trehalose, more preferably sodium chloride-trehalose.

The term "surfactant" generally includes agents that protect proteins, such as antibodies, from gas/liquid interface-induced stress and liquid/solid-induced stress to reduce aggregation of the antibodies or minimize the formation of particulate matters in the formulation. Exemplary surfactants include, but are not limited to, nonionic surfactants such as polyoxyethylene sorbitan fatty acid esters (e.g., polysorbate 20 and polysorbate 80), polyethylene-polypropylene copolymers, polyethylene-polypropylene glycol, polyoxyethylene stearate, polyoxyethylene alkyl ethers such as polyoxyethylene monolauryl ether, alkylphenyl polyoxyethylene ether (Triton-X), polyoxyethylene-polyoxypropylene copolymers (poloxamers, Pluronics) and sodium dodecyl sulfate (SDS).

The term "isotonic" refers to a formulation having substantially equivalent osmotic pressure to human blood. An isotonic formulation generally has an osmotic pressure of about 250 to 350 mOsm. Isotonicity can be measured by a vapor pressure osmometer or cryoscopic osmometer.

The term "stable" formulation is a formulation in which the antibody substantially retains its physical and/or chemical stability and/or bioactivity during the manufacturing process and/or upon storage. A pharmaceutical formulation can be stable even if the contained antibody fails to retain 100% of its chemical structure or biological function after a certain period of storage. In certain instances, a pharmaceutical formulation that retains about 90%, about 95%, about 96%, about 97%, about 98%, or about 99% of antibody structure or function after a certain period of storage may also be considered "stable". Various analytical techniques for measuring protein stability are available in the art and are described in Peptide and Protein Drug Delivery, 247-301, Vincent Lee ed., Marcel Dekker, Inc., New York, N.Y., Pubs. (1991) and Jones, A., (1993) Adv. Drug Delivery Rev., 10:29-90, both of which are incorporated herein for reference.

After a certain period of storage at a certain temperature, the stability of the formulation can be measured by determining the percentage of remaining native antibody (and by other methods). Except other methods, the percentage of native antibody can be measured by size-exclusion chromatography (e.g., size-exclusion high-performance liquid chromatography (SEC-HPLC)), and "native" refers to unaggregated and undegraded. In some embodiments, the stability of a protein is determined by a percentage of monomeric protein in a solution having a low percentage of degraded (e.g., fragmented) and/or aggregated protein. In some embodiments, the formulation is stable at room temperature, about 25-30 °C, or 40 °C for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months or longer, and the formulation has no more than about 6%, 5%, 4%, 3%, 2%, 1%, 0.5%, or 0.1% of antibody in aggregated form.

Stability can be measured by determining the percentage of migrating antibody in a fraction that is more acidic ("acidic form") than the main fraction of the antibody ("main charged form") during ion exchange (and can be measured by other methods), wherein the stability is inversely proportional to the percentage of acidic form of antibody. The percentage of "acidified" antibody can be measured by, except other methods, ion exchange chromatography (e.g., cation exchange high-performance liquid chromatography (CEX-HPLC)). In some embodiments, an acceptable stability means that the acidic form of the antibodies that can be detected in the formulation is no more than about 49%, 45%, 40%, 35%, 30%, 25%, 20%, 15%, 10%, 5%, 4%, 3%, 2%, 1%, 0.5% or 0.1% after a certain period of storage at a certain temperature. The certain period of storage prior to measuring stability can be at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When the stability is assessed, the certain temperature at which the pharmaceutical formulation is allowed to be stored may be any temperature in a range of about -80 °C to about 45 °C, e.g., at about -80 °C, about -30 °C, about -20 °C, about 0 °C, about 2-8 °C, about 5 °C, about 25 °C or about 40 °C.

An antibody in pharmaceutical composition "retains its physical stability" if the antibody does not substantially show signs of, such as aggregation, precipitation and/or denaturation, during visual inspection of color and/or clarity or by UV light scattering or measurement by pore-exclusion chromatography. Aggregation is a process in which individual molecules or complexes associate, covalently or non-covalently, to form aggregates. Aggregation can proceed to the point where a visible precipitate is formed.

Stability, e.g., physical stability, of a formulation can be assessed by methods well known in the art, including measuring the apparent extinction (absorbance or optical density) of a sample. Such extinction measurement correlates with the turbidity of the formulation. Turbidity of a formulation is, in part, an inherent property of proteins dissolved in a solution and is generally measured by nephelometry and is measured in nephelometric turbidity unit (NTU).

Turbidity levels that vary with, for example, the concentration of one or more components in a solution (e.g., protein and/or salt concentration) are also referred to "opacification" or "opacified appearance" of a formulation. Turbidity levels can be calculated with reference to a standard curve based on suspensions of known turbidity. The reference standards for determining the turbidity level of a pharmaceutical composition may be based on the standard of the European Pharmacopoeia, 4th edition, Directorate for the Quality of Medicine of the Council of Europe (EDQM), Strasbourg, France. According to the standard of *European Pharmacopoeia,* a clear solution is defined as a solution having a turbidity lower than or equal to that of a reference suspension according to the standard of *European Pharmacopoeia* having a turbidity of about 3. Turbidity measurement by nephelometry can measure Rayleigh scattering in the absence of association or non-ideal effects, which generally varies linearly with concentration. Other methods for assessing physical stability are well known in the art.

An antibody "retains its chemical stability" in a pharmaceutical composition if the chemical stability of the antibody at a given time point allows the antibody to retain its bioactivity as defined hereinafter. Chemical stability can be assessed, for example, by detecting or quantifying the form of chemical changes in the antibody. Chemical changes can include size changes (e.g., truncation), which can be assessed by, for example, size-exclusion chromatography, SDS-PAGE and/or matrix-assisted laser desorption/ionisation time-of-flight mass spectrometry (MALDI/TOF MS). Other types of chemical changes include charge changes (e.g., occurring as a result of deamidation or oxidation), which can be assessed by, for example, ion exchange chromatography.

An antibody in a pharmaceutical composition "retains its bioactivity" if it is biologically active for its intended purpose. For example, the formulation described herein is considered stable if, after a certain period of storage (e.g., 1 to 12 months) at a certain temperature, e.g., 5 °C, 25 °C or 45 °C, the binding affinity of the anti-BTLA antibody in the formulation for BTLA is at least 90%, 95% or greater of that of the antibody prior to storage. Binding affinity can also be determined by, for example, ELISA or plasmon resonance.

In the context of the present invention, a "therapeutically effective amount" or "effective amount" of an antibody, pharmacologically refers to an amount that is effective in preventing, treating or alleviating the symptoms of a disorder that the antibody can effectively treat. In the present invention, "therapeutically effective amount" or "therapeutically effective dose" of a medicament is any amount of the medicament that, when used alone or in combination with an additional therapeutic agent, protects a subject from the onset of a disease or promotes the regression of a disease as evidenced by a decrease in the severity of disease symptoms, an increase in the frequency and duration of disease symptom-free phase, or the prevention of injury or disability resulting from the affliction of the disease. The ability of a medicament to promote the regression of a disease can be assessed using a variety of methods known to those skilled in the art, such as in human subjects during clinical trials, in animal model systems that predict human efficacy, or by determining the activity of the agent in an *in vitro* assay. A therapeutically effective amount of a medicament includes a "prophylactically effective amount" which is any amount of a medicament that, when administered alone or in combination with other therapeutic drugs to a subject at risk of developing diseases or a subject having disease recurrence, inhibits the development or recurrence of diseases.

The term "subject" or "patient" is intended to include mammalian organisms. Examples of subjects/patients include human and non-human mammals such as non-human primates, dogs, cows, horses, pigs, sheep, goats, cats, mice, rabbits, rats and transgenic non-human animals. In one specific embodiment of the present invention, the subject is human.

The terms "administering", "giving" and "treating" refer to introducing a composition comprising a therapeutic agent into a subject using any one of a variety of methods or delivery systems known to those skilled in the art. Routes of administration of the anti-PD-1 antibody include intravenous, intramuscular, subcutaneous, intraperitoneal, spinal or other parenteral routes of administration, such as injection or infusion. "Parenteral administration" refers to modes of administration apart from enteral or local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion, and *in vivo* electroporation.

### Anti-BTLA antibody

The term "antibody" as used herein should be construed including intact antibody molecules and antigen-binding fragments thereof. The term "antigen-binding moiety" or "antigen-binding fragment" of an antibody (or simply "antibody moiety" or "antibody fragment") as used herein refers to one or more fragments of an antibody that retain the ability to specifically bind to human BTLA (B and T lymphocyte attenuator) or an epitope thereof.

The term "full-length antibody" or "intact antibody molecule" as used herein refers to an immunoglobulin molecule comprising four peptide chains, including two heavy (H) chains (about 50-70 kDa in total length) and two light (L) chains (about 25 kDa in total length) linked to each other by disulfide bonds. Each heavy chain consists of a heavy chain variable region (abbreviated herein as VH) and a heavy chain constant region (abbreviated herein as CH). The heavy chain constant region consists of 3 domains CHI, CH2 and CH3. Each light chain consists of a light chain variable region (abbreviated herein as VL) and a light chain constant region. The light chain constant region consists of one domain CL. The VH and VL regions can be further divided into complementarity determining regions (CDRs) with high variability and more conservative regions called framework regions (FRs) that are spaced apart by the CDRs. Each VH or VL region consists of 3 CDRs and 4 FRs arranged in the following order from the amino terminus to the carboxyl terminus: FR1, CDR1, FR2, CDR2, FR3, CDR3, FR4. The variable regions of the heavy and light chains contain binding domains that interact with antigens. The constant region of the antibody can mediate the binding of immunoglobulins to host tissues or factors, including the binding of various cells of the immune system (e.g., effector cells) to the first component (C1q) of classical complement system.

As used herein, the term "CDR" refers to the complementarity determining regions within an antibody variable sequence. There are 3 CDRs in each variable region of the heavy and light chains, which are named HCDR1, HCDR2 and HCDR3, and LCDR1, LCDR2 and LCDR3 for heavy and light chain variable regions. Exact boundaries of the CDRs may vary among different systems.

The precise amino acid sequence boundaries of the variable region CDRs of the antibodies disclosed herein can be determined using any of a number of well-known schemes, including Chothia based on the three-dimensional structure of antibodies and the topology of the CDR loops (Chothia et al., (1989) Nature 342:877-883; Al-Lazikani et al., "Standard conformations for the canonical structures of immunoglobulins", Journal of Molecular Biology, 273:927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (1999 Nucleic Acids Research, 27, 209-212), and North CDR definition based on the affinity propagation clustering using a large number of crystal structures. The boundaries of the CDRs of the antibodies disclosed herein can be determined by one skilled in the art according to any scheme (e.g., different assignment systems or combinations) in the art.

"Antigen-binding fragment" as used herein includes an antibody fragment or a derivative thereof, generally including at least one fragment of an antigen-binding region or variable region (e.g., one or more CDRs) of a parent antibody, which retains at least some of the binding specificity of the parent antibody. Examples of antigen-binding fragments include, but are not limited to, Fab, Fab', F(ab')₂ and Fv fragments; a diabody; a linear antibody; a single-chain antibody molecule, such as sc-Fv; a nanoclay and multispecific antibody formed by antibody fragments. A binding fragment or a derivative thereof generally retains at least 10% of the antigen-binding activity of the parent antibody when the binding activity of the antibody is expressed on a molar concentration basis. Preferably, the binding fragment or the derivative thereof retains at least 20%, 50%, 70%, 80%, 90%, 95% or 100% or more of the antigen-binding affinity of the parent antibody. It is also contemplated that an antigen-binding fragment of an antibody may include conservative or non-conservative amino acid substitutions that do not significantly alter its bioactivity (referred to as "conservative variants" or "function-conservative variants" of the antibody).

The anti-BTLA antibody or the antigen-binding fragment thereof described herein include any of the anti-BTLA antibody described in application No. CN201810870514.0, the disclosure of which is incorporated herein by reference in its entirety. In some embodiments, the CDR sequences of the antibody used in the methods and compositions of the present invention comprise the CDR sequences of antibody hu18 described in CN201810870514.0. In some embodiments, the CDR sequences of the antibody used in the methods and compositions of the present invention comprise the CDR sequences of antibody hu17 described in CN201810870514.0. In some embodiments, the CDR sequences of the antibody used in the methods and compositions of the present invention comprise the CDR sequences of antibody hu19 described in CN201810870514.0.

The non-limiting and exemplary antibody used in the examples herein is selected from hu17, hu18 and hu19 described in CN201810870514.0, which are all full humanized antibodies that specifically bind to human BTLA, wherein antibodies hu17, hu18 and hu19 all have amino acid sequences an HCDR1, an HCDR2 and an HCDR3 set forth in SEQ ID NO: 1, SEQ ID NO: 2 and SEQ ID NO: 3, respectively, and amino acid sequences an LCDR1, an LCDR2 and an LCDR3 set forth in SEQ ID NO: 4, SEQ ID NO: 5 and SEQ ID NO: 6, respectively; preferably, antibody hu17 has a heavy chain variable region having an amino acid sequence set forth in SEQ ID NO: 7 and a light chain variable region having an amino acid sequence set forth in SEQ ID NO: 8; the antibody hu18 has a heavy chain variable region having an amino acid sequence set forth in SEQ ID NO: 7 and a light chain variable region having an amino acid sequence set forth in SEQ ID NO: 9; the antibody hu19 has a heavy chain variable region having an amino acid sequence set forth in SEQ ID NO: 7 and a light chain variable region having an amino acid sequence set forth in SEQ ID NO: 10; preferably, antibody hu18 has a heavy chain amino acid sequence set forth in SEQ ID NO: 11 and a light chain amino acid sequence set forth in SEQ ID NO: 12. The CDR amino acid sequences of the above humanized antibody are defined by the IMGT system.

### Pharmaceutical formulation

The pharmaceutical composition provided by the present invention is a highly stable pharmaceutical composition containing a humanized antibody specifically binding to BTLA. In particular, the present inventors have found that the combination of trehalose with sodium chloride may significantly improve the stability of the pharmaceutical composition.

The present invention provides a pharmaceutical composition comprising: (1) a buffer; and (2) an anti-BTLA antibody or an antigen-binding fragment thereof.

The antibody in the pharmaceutical composition of the present invention may be a murine antibody, a chimeric antibody and a humanized antibody, preferably a humanized antibody, and may have an HCDR1, an HCDR2 and an HCDR3 set forth in SEQ ID NO: 1, SEQ ID NO: 2 and SEQ ID NO: 3, respectively, and an LCDR1, an LCDR2 and an LCDR3 set forth in SEQ ID NO: 4, SEQ ID NO: 5 and SEQ ID NO: 6, respectively. Preferably, the antibody in the pharmaceutical composition of the present invention has a heavy chain variable region set forth in SEQ ID NO: 7 and a light chain variable region set forth in SEQ ID NO: 8; or has a heavy chain variable region set forth in SEQ ID NO: 7 and a light chain variable region set forth in SEQ ID NO: 9; or has a heavy chain variable region set forth in SEQ ID NO: 7 and a light chain variable region set forth in SEQ ID NO: 10; more preferably, the antibody in the pharmaceutical composition of the present invention has a heavy chain amino acid sequence set forth in SEQ ID NO: 11 and a light chain amino acid sequence set forth in SEQ ID NO: 12.

The anti-BTLA antibody or the antigen-binding fragment thereof in the pharmaceutical composition of the present invention has a concentration of about 1-200 mg/mL, preferably about 5-100 mg/mL, preferably about 10-50 mg/mL, and more preferably 15-25 mg/mL. As non-limiting examples, the anti-BTLA antibody or the antigen-binding fragment thereof has a concentration of about 10 mg/mL, 15 mg/mL, 20 mg/mL, 25 mg/mL, 30 mg/mL, 35 mg/mL, 40 mg/mL, 45 mg/mL or 50 mg/mL; preferably about 10 mg/mL, 20 mg/mL or 40 mg/mL.

The buffer in the pharmaceutical composition of the present invention may be selected from an acetic acid buffer, a citric acid buffer and a histidine buffer to provide a pH of 5.0 to 6.5, preferably 5.0 to 6.0, to the pharmaceutical composition of the present invention. In another aspect, the buffer used in the pharmaceutical composition of the present invention may have a pH of 5.0 to 6.5, preferably 5.0 to 6.0.

A particularly preferred buffer in the pharmaceutical composition of the present invention is a histidine buffer. Preferably, the histidine buffer used in the present invention has a pH of 5.0 to 6.0, more preferably 5.5 ± 0.3, preferably about 5.5. Preferably, the histidine buffer comprises a histidine-hydrochloride buffer or a histidine-acetate buffer, preferably a histidine-hydrochloride buffer. More preferably, the histidine-hydrochloride buffer consists of histidine and histidine hydrochloride, preferably L-histidine and L-histidine monohydrochloride. In some embodiments, the histidine buffer is prepared by 1-20 mM L-histidine and 1-20 mM L-histidine monohydrochloride. In some embodiments, the histidine buffer consists of histidine and histidine hydrochloride in a molar ratio of 1:1-1:4. In some embodiments, the histidine buffer consists of histidine and histidine hydrochloride in a molar ratio of 1:1. In some embodiments, the histidine buffer consists of histidine and histidine hydrochloride in a molar ratio of 1:3. In some embodiments, the histidine formulation is a histidine buffer at pH 5.5 prepared by 4.5 mM L-histidine and 15.5 mM L-histidine monohydrochloride. In some embodiments, the histidine formulation is a histidine buffer at pH 6.0 prepared by 15 mM L-histidine and 15 mM L-histidine monohydrochloride.

The histidine buffer in the pharmaceutical composition of the present invention may be a histidine-acetic acid buffer preferably in a molar ratio of 1:1 to 1.5:1; preferably such a buffer has a pH of 5.5 ± 0.3, preferably about 5.5; preferably such a buffer contains 15-20 mM histidine and 12-15 mM acetic acid.

Accordingly, the pharmaceutical composition of the present invention may comprise: a histidine-histidine hydrochloride buffer at a pH of 5.0-6.0 and with a concentration of 10-30 mM in the pharmaceutical composition; and 15-25 mg/mL of the anti-BTLA antibody or the antigen-binding fragment thereof of any one of the embodiments above, particularly hu17, hu18, or hu19 or antigen-binding fragment thereof described herein.

In some embodiments, the pharmaceutical composition of the present invention further comprises a stabilizer. Preferably, the stabilizer is selected from one or more of sodium chloride, mannitol, sorbitol, sucrose, maltose, xylitol and trehalose. Preferably, the stabilizer in the pharmaceutical composition comprises at least sodium chloride, optionally one or more of mannitol, sorbitol, sucrose and trehalose. For example, the pharmaceutical composition may comprise sodium chloride and mannitol, sodium chloride and sorbitol, sodium chloride and sucrose, or sodium chloride and trehalose. When comprising the above compositions, the stabilizer in the pharmaceutical composition has a concentration of about 20 mM to 300 mM, preferably 50 mM to 300 mM, and more preferably 120 mM to 250 mM. In some embodiments, the stabilizer is sodium chloride at a concentration of about 50-200 mM; or the stabilizer is mannitol at a concentration of about 100-300 mM; or the stabilizer is sorbitol at a concentration of about 100-300 mM; or the stabilizer is sucrose at a concentration of about 100-300 mM; or the stabilizer is trehalose at a concentration of about 100-300 mM; or the stabilizer is a combination of about 30-100 mM sodium chloride and about 50-200 mM mannitol; the stabilizer is a combination of about 30-100 mM sodium chloride and about 50-200 mM sorbitol; the stabilizer is a combination of about 30-100 mM sodium chloride and about 50-200 mM sucrose; the stabilizer is a combination of about 30-100 mM sodium chloride and about 50-200 mM trehalose.

Thus, in some embodiments, the pharmaceutical composition of the present invention comprises: a histidine-histidine hydrochloride buffer at a pH of 5.0-6.0 and with a concentration of 10-30 mM in the pharmaceutical composition; 15-25 mg/mL of the anti-BTLA antibody or the antigen-binding fragment thereof of any one of the embodiments, particularly hu17, hu18, or hu19 or antigen-binding fragment thereof described herein; and 20-300 mM stabilizer, preferably, the stabilizer at least comprises sodium chloride, optionally comprises one of mannitol, sorbitol, sucrose and trehalose, preferably 50-150 mM sodium chloride, or 40-80 mM sodium chloride and 120-150 mM mannitol, or 40-80 mM sodium chloride and 120-150 mM sorbitol, or 40-80 mM sodium chloride and 120-150 mM sucrose, or 40-80 mM sodium chloride and 120-150 mM of trehalose. In some embodiments, the stabilizer is 200-300 mM trehalose.

In some embodiments, the pharmaceutical composition of the present invention further comprises a surfactant. The preferred surfactant is selected from polysorbate 80, polysorbate 20 and poloxamer 188. The most preferred surfactant is polysorbate 80. The surfactant in the pharmaceutical composition of the present invention has a concentration of about 0.001%-0.1%, preferably about 0.01%-0.05%, and preferably about 0.02%-0.04% (w/v). As non-limiting examples, the surfactant in the pharmaceutical composition of the present invention has a concentration of about 0.02%, 0.03% or 0.04%, preferably 0.02%.

Thus, in some embodiments, the pharmaceutical composition of the present invention comprises: a histidine-histidine hydrochloride buffer at a pH of 5.0-6.0 and with a concentration of 10-30 mM in the pharmaceutical composition; 15-25 mg/mL of the anti-BTLA antibody or the antigen-binding fragment thereof of any one of the embodiments, particularly hu17, hu18, or hu19 or antigen-binding fragment thereof described herein; 20-300 mM stabilizer, preferably, the stabilizer is trehalose, or at least comprises sodium chloride, optionally comprises one of mannitol, sorbitol, sucrose and trehalose, preferably 200-300 mM trehalose, or 50-150 mM sodium chloride, or 40-80 mM sodium chloride and 120-150 mM mannitol, or 40-80 mM sodium chloride and 120-150 mM sorbitol, or 40-80 mM sodium chloride and 120-150 mM sucrose, or 40-80 mM sodium chloride and 120-150 mM trehalose; and 0.02%-0.04% (w/v) of polysorbate 80. In a particularly preferred embodiment, the pharmaceutical composition of the present invention comprises or consists of: a histidine-histidine hydrochloride buffer at a pH of 5.5 ± 0.3 and with a concentration of 20 ± 5 mM; 40-80 mM sodium chloride; 120-150 mM trehalose, 0.02-0.04% (w/v) of polysorbate 80; 15-25 mg/mL of an antibody hu18 or an antigen-binding fragment thereof.

The pharmaceutical composition of the present invention may be a liquid formulation or a lyophilized formulation. It should be understood that the liquid formulation, in addition to the buffers, stabilizers, antibodies or antigen-binding fragments thereof and surfactants described herein, also comprises water for formulating the pharmaceutical composition.

### Medical uses and methods

The present invention also provides the pharmaceutical composition according to any embodiment of the present invention for use in treating or preventing BTLA-mediated diseases, use of the pharmaceutical composition according to any embodiment of the present invention in the preparation of a medicament for treating or preventing BTLA-mediated diseases, and a method for administering a therapeutically effective amount of the pharmaceutical composition according to any embodiment of the present invention to a subject or patient in need thereof to treat or prevent BTLA-mediated diseases.

In the present invention, the BTLA-mediated diseases refer to diseases in which BTLA is involved in the development and progression of diseases, including, but not limited to, tumors, infectious diseases, and inflammatory or autoimmune diseases. In the present invention, tumors suitable for treatment and prevention with the pharmaceutical composition of the present invention include melanoma, breast cancer, renal cancer, prostate cancer, colon cancer, lung cancer, pancreatic cancer, bone cancer, skin cancer, head or neck cancer, uterine cancer, ovarian cancer, rectal cancer, anal cancer, stomach cancer, testicular cancer, small intestine cancer, cervical cancer, vaginal cancer, Hodgkin's disease, non-Hodgkin's lymphoma, esophageal cancer, cancer of the endocrine system, thyroid cancer, carcinoma of adrenal gland, soft tissue cancer, urethral cancer, chronic or acute leukemia, chronic myeloid leukemia, acute lymphoblastic leukemia, chronic lymphocytic leukemia, childhood solid tumor, lymphocytic lymphoma, bladder cancer, kidney or ureteral cancer, renal pelvis cancer, neoplasms of the central nervous system, primary central nervous system lymphoma, tumor angiogenesis, spinal axis tumor, brain stem glioma, pituitary adenoma, Kaposi's sarcoma, epidermoid carcinoma, squamous cell carcinoma, T-cell lymphoma, environmentally induced cancers including those induced by asbestos, and combinations of the cancers. The autoimmune diseases suitable for treatment and prevention with the pharmaceutical composition of the present invention include organ-specific autoimmune diseases and systemic autoimmune diseases; the organ-specific autoimmune diseases include chronic lymphocytic thyroiditis, hyperthyroidism, insulin-dependent diabetes mellitus, myasthenia gravis, ulcerative colitis, pernicious anemia with chronic atrophic gastritis, goodpasture's syndrome, pemphigus vulgaris, pemphigoid, primary biliary cirrhosis, multiple sclerosis, acute idiopathic polyneuritis and the like; and the systemic autoimmune diseases include systemic lupus erythematosus, rheumatoid arthritis, systemic vasculitis, scleroderma, pemphigus, dermatomyositis, mixed connective tissue disease, autoimmune hemolytic anemia, ulcerative colitis and the like.

### DETAILED DESCRIPTION

The present invention will be illustrated hereinafter by way of specific examples. It should be understood that these examples are illustrative only and are not intended to limit the scope of the present invention. The methods and materials used in the examples are, unless otherwise indicated, conventional in the art.

### Example 1: Buffer System and pH Selection

In a liquid pharmaceutical composition, the buffer system and pH closely affect the stability of the antibody, and each antibody having unique physicochemical properties may have an optimum buffer and pH. This example is intended to select an optimal buffer system and pH to provide the anti-BTLA antibody disclosed herein with optimal stability for clinical application.

This example was performed with antibody hu18 at concentrations of about 10 mg/mL, 20 mg/mL and 40 mg/mL. Buffer concentration and exchange by ultrafiltration was performed on the samples using a VIVAFlow200, the samples after buffer exchange were added into a corresponding formula, and the samples were sealed in a centrifuge tube for buffer selection. An acetic acid buffer, a citric acid buffer and a histidine buffer were selected at a pH from 5.0 to 6.5 (as shown in Table 1). The samples were placed at 40 °C and removed for analysis at weeks 0, 2 and 4. The major degradation pathways of the protein were the formations of aggregates, cleavage products and charged variants. The percentages of native (protein monomer) and aggregated protein monomer were determined by size exclusion high-performance liquid chromatography (SEC-HPLC) and the percentages of acidic and basic antibody were determined by cation exchange high-performance chromatography (CEX-HPLC). The effect of different buffer systems and pH on the antibody hu18 stability was examined at baseline (0 w), two weeks (2 w) and four weeks (4 w). By linear fitting, straight lines were plotted according to the SEC-HPLC monomer content and the CEX-HPLC main peak content and the slopes (%/week) were calculated. The results are summarized in Table 2 and Table 3.

**Table 1. Formula for buffer system and pH selection**

| Formula No. | pH | Buffer system | Excipient 1 | Excipient 2 |
|---|---|---|---|---|
| 1 | 5.0 | 20 mM acetic acid buffer (acetic acid-sodium acetate) | 50 mM sodium chloride | 140 mM mannitol |
| 2 | 5.0 | 20 mM acetic acid buffer (acetic acid-sodium acetate) | 140 mM sodium chloride | - |
| 3 | 5.5 | 20 mM histidine buffer (histidine-acetic acid) | 50 mM sodium chloride | 140 mM mannitol |
| 4 | 5.5 | 20 mM histidine buffer (histidine-acetic acid) | 140 mM sodium chloride | - |
| 5 | 5.5 | 20 mM acetic acid buffer (acetic acid-sodium acetate) | 50 mM sodium chloride | 140 mM mannitol |
| 6 | 5.5 | 20 mM acetic acid buffer (acetic acid-sodium acetate) | 140 mM sodium chloride | - |
| 7 | 6.0 | 20 mM histidine buffer (histidine-histidine hydrochloride) | 50 mM sodium chloride | 140 mM mannitol |
| 8 | 6.0 | 20 mM histidine buffer (histidine-histidine hydrochloride) | 140 mM sodium chloride | - |
| 9 | 6.0 | 20 mM citric acid buffer (citric acid-sodium citrate) | 50 mM sodium chloride | 140 mM mannitol |
| 10 | 6.0 | 20 mM citric acid buffer (citric acid-sodium citrate) | 140 mM sodium chloride | - |
| 11 | 6.5 | 20 mM citric acid buffer (citric acid-sodium citrate) | 50 mM sodium chloride | 140 mM mannitol |
| 12 | 6.5 | 20 mM citric acid buffer (citric acid-sodium citrate) | 140 mM sodium chloride | - |

| | | | | |
|---|---|---|---|---|
| Note: "-" represents no addition. | | | | |

**Table 2. Degradation rate of SEC-HPLC monomer content in buffer system and pH selection**

| Formula No. | 10 mg/mL of protein Monomer decline (%/week) | 20 mg/mL of protein Monomer decline (%/week) | 40 mg/mL of protein Monomer decline (%/week) | Average decline of monomer (%/week) |
|---|---|---|---|---|
| 1 | 0.27 | 0.38 | 0.52 | 0.39 |
| 2 | 0.46 | 0.53 | 0.71 | 0.57 |
| 3 | 0.17 | 0.27 | 0.38 | 0.27 |
| 4 | 0.23 | 0.36 | 0.48 | 0.36 |
| 5 | 0.22 | 0.32 | 0.46 | 0.34 |
| 6 | 0.36 | 0.45 | 0.58 | 0.46 |
| 7 | 0.16 | 0.21 | 0.35 | 0.24 |
| 8 | 0.14 | 0.24 | 0.37 | 0.25 |
| 9 | 0.23 | 0.33 | 0.44 | 0.33 |
| 10 | 0.21 | 0.31 | 0.41 | 0.31 |
| 11 | 0.27 | 0.37 | 0.52 | 0.38 |
| 12 | 0.24 | 0.35 | 0.46 | 0.35 |

**Table 3. Degradation rate of CEX-HPLC charge heteroplasmon content in buffer system and pH selection**

| Formula No. | 10 mg/mL of protein Main peak decline (%/week) | 20 mg/mL of protein Main peak decline (%/week) | 40 mg/mL of protein Main peak decline (%/week) | Average decline of major peak (%/week) |
|---|---|---|---|---|
| 1 | 5.82 | 5.44 | 5.38 | 5.54 |
| 2 | 5.48 | 4.67 | 4.89 | 5.01 |
| 3 | 6.08 | 4.65 | 4.45 | 5.06 |
| 4 | 7.75 | 5.89 | 5.87 | 6.51 |
| 5 | 6.46 | 5.00 | 4.57 | 5.34 |
| 6 | 6.88 | 4.75 | 3.51 | 5.05 |
| 7 | 6.67 | 6.35 | 5.50 | 6.17 |
| 8 | 6.58 | 6.85 | 5.53 | 6.32 |
| 9 | 7.11 | 5.27 | 6.69 | 6.36 |
| 10 | 7.06 | 5.11 | 5.67 | 5.95 |
| 11 | 8.46 | 8.23 | 7.11 | 7.93 |
| 12 | 6.52 | 5.64 | 4.84 | 5.67 |

As shown in Tables 2 and 3, in SEC-HPLC analysis, after the monomer was placed at a high temperature of 40 °C for 4 weeks, the monomer content declined at a high rate along with the increase of the protein concentration, while the monomer content declined at a low rate under a histidine buffer system with a pH of 5.5-6.0, and the average decline was less than or equal to 0.27%/week; in CEX-HPLC analysis, it could be seen that the main peak content degradation rate by CEX-HPLC had a certain correlation with pH, the higher the pH was, the higher the main peak content degradation rate was, the main peak content degradation rate was relatively low in a buffer system with a pH of 5.0-6.0, and the correlation between the main peak content degradation rate and the protein concentration was not significant.

After the product specification and the target quality attribute (polymer content level) of the product were comprehensively considered, a histidine buffer with the protein concentration of 20 mg/mL and a pH of 5.5-6.0 was selected for the selection of excipient (stabilizer) formula.

### Example 2: Stabilizer Selection

In order to further explore the effect of different excipients on the stability of the antibody, a formulation of one or a combination of sodium chloride, mannitol, sorbitol, sucrose or trehalose was selected for comparative testing. The above various excipients or combinations thereof were added into 20 mM histidine buffer or citric acid buffer comprising about 20 mg/mL of antibody hu18, and the specific formula is shown in Table 4. The formulations were aliquoted, then placed at 40 °C, and removed for analysis at weeks 0, 2 and 4. Changes in antibody hu18 content were determined by size-exclusion high-performance liquid chromatography (SEC-HPLC), and the charged main peak content of antibody hu18 was determined by cation exchange high-performance liquid chromatography (CEX-HPLC). The results are shown in Table 5.

According to the results of stability investigation, after the formulation samples of different excipients were placed at a high temperature of 40 °C for 2 weeks, the antibody had stronger thermostability.

After comprehensive data analysis, it could be found from comparing F13-F17 with F18-F21 that the excipient combination of mannitol, sorbitol, sucrose, trehalose and sodium chloride was better than a single excipient of mannitol, sorbitol, sucrose and trehalose, which was manifested in that the degradation rates of the monomer content by SEC-HPLC and the main peak content by CEX-HPLC were lower. In the excipient comprising trehalose, a histidine buffer system with a pH of 5.5 was the most stable, the decline of antibody monomer purity was as low as 0.11%/week, and the decline of antibody main charge was only 3.72%/week, which was significantly lower than those in a buffer system with a pH of 6.0.

Therefore, the excipient combination of the trehalose and the sodium chloride with a pH of 5.5 was more favorable for the stability of the product. It could also be seen that a histidine buffer system with a pH of 6.0 (F17) was significantly better than a citrate buffer system with a pH of 6.0 (F23) for degradation of the monomer content by SEC-HPLC.

**Table 4. Formulas in excipient selection**

| Formula No. | Buffer | PH | Excipient 1 | Excipient 2 | Polysorbate 80 |
|---|---|---|---|---|---|
| 13 | 20 mM histidine buffer (histidine-histidine hydrochloride) | 6.0 | 135 mM sodium chloride | - | 0.02% |
| 14 | | | 240 mM mannitol | - | 0.02% |
| 15 | | | 240 mM sorbitol | - | 0.02% |
| 16 | | | 220 mM sucrose | - | 0.02% |
| 17 | | | 220 mM trehalose | - | 0.02% |
| 18 | | | 54 mM sodium chloride | 144 mM mannitol | 0.02% |
| 19 | | | 54 mM sodium chloride | 144 mM sorbitol | 0.02% |
| 20 | | | 54 mM sodium chloride | 132 mM sucrose | 0.02% |
| 21 | | | 54 mM sodium chloride | 132 mM trehalose | 0.02% |
| 22 | 20 mM histidine buffer (histidine-acetic acid) | 5.5 | 220 mM trehalose | - | 0.02% |
| 23 | 20 mM citric acid buffer (citric acid-sodium citrate) | 6.0 | 220 mM trehalose | - | 0.02% |

| | | | | | |
|---|---|---|---|---|---|
| Note: "-" represents no addition. | | | | | |

**Table 5. Summary of results of excipient selection**

| Formula No. | SEC-HPLC Monomer decline (%/week) | CEX-HPLC Main peak decline (%/week) |
|---|---|---|
| 13 | 0.13 | 4.66 |
| 14 | 0.12 | 5.11 |
| 15 | 0.12 | 5.13 |
| 16 | 0.12 | 5.08 |
| 17 | 0.13 | 5.27 |
| 18 | 0.12 | 4.98 |
| 19 | 0.11 | 4.92 |
| 20 | 0.12 | 4.99 |
| 21 | 0.12 | 4.81 |
| 22 | 0.11 | 3.72 |
| 23 | 0.19 | 4.99 |

### Example 3: Surfactant Selection

Surfactants are commonly used in liquid formulations as an agent for protecting proteins such as antibodies from gas/liquid-induced stress, liquid/solid-induced stress during storage to reduce aggregation of the antibodies or minimize the formation of particulate matter in the formulation, which facilitates the stability of the physicochemical properties of the antibodies. Polysorbate 20 or polysorbate 80 at different concentrations was added into the formulations comprising 20 mM histidine buffer ( in a molar ratio of histidine to histidine hydrochloride of 1:1, pH 6.0) and 20 mg/mL of antibody hu18, and the mixture was analyzed after placed at 40 °C for 4 weeks. The results are shown in Table 6.

After comprehensive data analysis, it was shown from the selection of surfactants (F17, F24, F25 and F26) that the addition of different concentrations of polysorbate 80 or polysorbate 20 had no significant effect on the monomer content by SEC-HPLC and the main peak content by CEX-HPLC.

**Table 6. Results of surfactant selection**

| Formula No. | Excipient | Surfactant content | SEC-HPLC (%) Monomer (%) | CEX-HPLC (%) Main charged form (%) |
|---|---|---|---|---|
| 17 | 220 mM trehalose | 0.02% of polysorbate 80 | 0.13 | 5.27 |
| 24 | | 0.04% of polysorbate 80 | 0.13 | 4.95 |
| 25 | | 0.02% of polysorbate 20 | 0.12 | 4.98 |
| 26 | | 0.04% of polysorbate 20 | 0.13 | 4.99 |

In summary, the stability of the recombinant humanized anti-BTLA monoclonal antibody hu18 is explored and researched by examining different buffer systems, different pH conditions, different antibody concentrations and different excipients and surfactant compositions, and the formula of optimal liquid formulation is determined. A histidine buffer was selected for adjusting the pH of antibody hu18, trehalose and sodium chloride were selected for adjusting the osmotic pressure of formulation, and polysorbate 80 was added to increase the solubility of the formulation.

### Example 4: Long-term Stability Study

Liquid pharmaceutical products comprising therapeutic antibodies generally require a temperature of 2-8 °C for storage, which is important for the stability of the formulation over a long-term storage. Based on the above selection results, formula No. 27 on the basis of the previous 26 formulas was designed to perform long-term stability study of the formulation.

Four batches of stock solutions were selected for formulating the formulations of formula No. 27 as shown in Table 7, then the formulations were stored in transparent vials, and the formulation samples were analyzed after placed at 2-8 °C for 6 months. Stability was assessed by the following parameters: (a) appearance; (b) insoluble microparticles by light-blockage method (OD: 405 nm); (c) pH; (d) molecular weight of the antibody by CE-SDS (capillary electrophoresis sodium dodecyl sulfate); (d) contents of antibody monomer (specification: ≥ 97.0%), polymers (specification: ≤ 3.0%) or fragments (specification: ≤ 1.0%) by SEC-HPLC; (e) main charged form (specification: ≥ 70.0%), acidic forms (specification: ≤ 30.0%) or basic forms (specification: ≤ 15.0%) of the antibody by CEX-HPLC; (f) antibody binding activity by ELISA (specification: 70%-130% of reference standard); and (g) protein content (specification: 18-22 mg/mL).

The results showed that the appearance, pH, insoluble microparticles, protein content, purity (determined by SEC-HPLC (size-exclusion high-performance liquid chromatography), CEX-HPLC (cation exchange high-performance liquid chromatography), R-CE-SDS (reduced electrophoresis) and NR-CE-SDS (non-reduced electrophoresis)) and biological activity of 4 batches of stock solutions in the formulations of formula No. 27 were not significantly changed, and the specific results are shown in Table 8. The results show that the stock solutions of the 4 batches in the formulations of formula No. 27 have very good stability during storage for 0-24 months at 2-8 °C.

**Table 7. Formula for long-term stability study of formulation**

| Formula No. | pH | Composition |
|---|---|---|
| 27 | 5.5 | 20 mM histidine buffer, 50 mM sodium chloride, 140 mM trehalose, 0.02% of polysorbate 80 and 20 mg/mL of antibody hu18 |

| | | |
|---|---|---|
| Note: the histidine buffer is prepared by a molar ratio of histidine to histidine hydrochloride being 1:3. | | |

**Table 8. Long-term stability of formulations**

| Test item | Time (mont h) | Result | | | |
|---|---|---|---|---|---|
| | | Batch 1 | Batch 2 | Batch 3 | Batch 4 |
| Appearance | 0 | Colorless and clear liquid with slight opalescence | Colorless and clear liquid with slight opalescence | Colorless and clear liquid with slight opalescence | Colorless and clear liquid with slight opalescence |
| | 1 | Colorless and clear liquid with slight opalescence | NA | NA | Colorless and clear liquid with slight opalescence |
| | 2 | Colorless and clear liquid with slight opalescence | NA | NA | NA |
| | 3 | Colorless and clear liquid with slight opalescence | Colorless and clear liquid with slight opalescence | Colorless and clear liquid with slight opalescence | Colorless and clear liquid with slight opalescence |
| | 6 | Colorless and clear liquid with slight opalescence | Colorless and clear liquid with slight opalescence | Colorless and clear liquid with slight opalescence | Colorless and clear liquid with slight opalescence |
| | 9 | Colorless and clear liquid with slight opalescence | Colorless and clear liquid with slight opalescence | Colorless and clear liquid with slight opalescence | Colorless and clear liquid |
| | 12 | Colorless and clear liquid with slight opalescence | Colorless and clear liquid with slight opalescence | Colorless and clear liquid with slight opalescence | Colorless and almost clear liquid |
| | 18 | Colorless and clear liquid with slight opalescence | Colorless and clear liquid with slight opalescence | Colorless and clear liquid with slight opalescence | NR |
| | 24 | Colorless and clear liquid with slight opalescence | Colorless and clear liquid with slight opalescence | Colorless and clear liquid with slight opalescence | NR |
| pH | 0 | 5.6 | 5.6 | 5.6 | 5.7 |
| | 1 | 5.7 | NA | NA | 5.6 |
| | 2 | 5.6 | NA | NA | NA |
| | 3 | 5.6 | 5.4 | 5.4 | 5.6 |
| | 6 | 5.6 | 5.6 | 5.6 | 5.7 |
| | 9 | 5.6 | 5.6 | 5.6 | 5.9 |
| | 12 | 5.6 | 5.7 | 5.6 | 5.6 |
| | 18 | 5.6 | 5.6 | 5.6 | NR |
| | 24 | 5.6 | 5.6 | 5.6 | NR |
| SEC-HPLC monomer content (%) | 0 | 99.7% | 99.7% | 99.7% | 99.7% |
| | 1 | 99.7% | NA | NA | 99.7% |
| | 2 | 99.7% | NA | NA | NA |
| | 3 | 99.5% | 99.6% | 99.6% | 99.7% |
| | 6 | 99.7% | 99.6% | 99.6% | 99.6% |
| | 9 | 99.7% | 99.6% | 99.6% | 99.6% |
| | 12 | 99.7% | 99.5% | 99.5% | 99.6% |
| | 18 | 99.7% | 99.6% | 99.5% | NR |
| | 24 | 99.6% | 99.5% | 99.5% | NR |
| SEC-HPLC polymer content (%) | 0 | 0.3% | 0.3% | 0.0% | 0.3% |
| | 1 | 0.3% | NA | NA | 0.3% |
| | 2 | 0.3% | NA | NA | NA |
| | 3 | 0.5% | 0.4% | 0.0% | 0.3% |
| | 6 | 0.3% | 0.4% | 0.0% | 0.4% |
| | 9 | 0.3% | 0.4% | 0.4% | 0.4% |
| | 12 | 0.3% | 0.4% | 0.5% | 0.4% |
| | 18 | 0.3% | 0.4% | 0.5% | NR |
| | 24 | 0.3% | 0.4% | 0.5% | NR |
| SEC-HPLC fragment content (%) | 0 | 0.0% | 0.3% | 0.0% | 0.0% |
| | 1 | 0.0% | NA | NA | 0.0% |
| | 2 | 0.0% | NA | NA | NA |
| | 3 | 0.0% | 0.4% | 0.0% | 0% |
| | 6 | 0.0% | 0.4% | 0.0% | 0% |
| | 9 | 0.0% | <0.1% | <0.1% | <0.1% |
| | 12 | 0.0% | <0.1% | <0.1% | <0.1% |
| | 18 | 0.0% | <0.1% | <0.1% | NR |
| | 24 | <0.1% | <0.1% | <0.1% | NR |
| | 0 | 63.7% | 62.6% | 61.7% | 63.1% |
| | 1 | 61.5% | NA | NA | 62.6% |
| CEX-HPLC main peak content (%) | 2 | 61.7% | NA | NA | NA |
| | 3 | 61.1% | 65.4% | 64.6% | 64.3% |
| | 6 | 65.8% | 67.5% | 66.8% | 66.7% |
| | 9 | 65.5% | 68.8% | 68.2% | 68.5% |
| | 12 | 68.1% | 71.2% | 70.7% | 69.3% |
| | 18 | 70.4% | 70.8% | 70.1% | NR |
| | 24 | 69.9% | 71.5% | 71.1% | NR |
| CEX-HPLC acidic peak content (%) | 0 | 10.0% | 13.1% | 12.6% | 12.0% |
| | 1 | 11.0% | NA | NA | 13.0% |
| | 2 | 10.9% | NA | NA | NA |
| | 3 | 11.8% | 13.9% | 13.6% | 13.3% |
| | 6 | 12.4% | 14.2% | 13.7% | 15.2% |
| | 9 | 15.3% | 14.5% | 14.0% | 15.7% |
| | 12 | 13.1% | 14.5% | 14.1% | 16.0% |
| | 18 | 13.6% | 17.3% | 16.9% | NR |
| | 24 | 15.3% | 17.8% | 17.6% | NR |
| CEX-HPLC basic peak content (%) | 0 | 26.3% | 24.3% | 25.7% | 24.9% |
| | 1 | 27.5% | NA | NA | 24.4% |
| | 2 | 27.4% | NA | NA | NA |
| | 3 | 27.1% | 20.6% | 21.8% | 22.3% |
| | 6 | 21.8% | 18.3% | 20.0% | 18.1% |
| | 9 | 19.2% | 16.7% | 17.8% | 15.9% |
| | 12 | 18.7% | 14.2% | 15.2% | 14.8% |
| | 18 | 16.0% | 11.8% | 13.0% | NR |
| | 24 | 14.8% | 10.7% | 11.3% | NR |
| R-CE-SDS content (%) | 0 | 99.9% | 99.8% | 99.7% | 99.6% |
| | 1 | 99.8% | NA | NA | 99.5% |
| | 2 | 99.8% | NA | NA | NR |
| | 3 | 99.9% | 99.5% | 99.5% | 99.4% |
| | 6 | 99.9% | 99.4% | 99.4% | 99.6% |
| | 9 | 99.7% | 99.2% | 99.3% | 99.5% |
| | 12 | 99.6% | 99.2% | 99.2% | NR |
| | 18 | 99.6% | 99.3% | 99.2% | NR |
| | 24 | 99.2% | 99.4% | 99.3% | NR |
| NR-CE-SDS content (%) | 0 | 99.1% | 98.4% | 99.3% | 96.4% |
| | 1 | 99.0% | NA | NA | 97.5% |
| | 2 | 99.1% | NA | NA | NR |
| | 3 | 98.7% | 98.7% | 98.4% | 97.4% |
| | 6 | 98.0% | 97.8% | 97.8% | 93.8% |
| | 9 | 98.6% | 97.5% | 97.6% | 97.2% |
| | 12 | 98.2% | 97.5% | 97.2% | NR |
| | 18 | 98.2% | 98.2% | 98.1% | NR |
| | 24 | 98.4% | 96.9% | 96.8% | NR |
| Biological activity (by ELISA) % | 0 | 107.4% | 101% | 100% | 98% |
| | 1 | NA | NA | NA | 99% |
| | 2 | NA | NA | NA | NR |
| | 3 | NA | 95% | 100% | 101% |
| | 6 | 106.1% | 100% | 102% | 105% |
| | 9 | NA | 96% | 99% | 103% |
| | 12 | 109.7% | 95% | 100% | NR |
| | 18 | NA | 100% | 98% | NR |
| | 24 | 113% | 103% | 105% | NR |
| Protein content (ultraviolet spectrophotomet ry) | 0 | 21.3 mg/mL | 20.9 mg/mL | 20.9 mg/mL | 20.3mg/mL |
| | 1 | 21.8 mg/mL | NA | NA | 20.5mg/mL |
| | 2 | 21.6 mg/mL | NA | NA | NA |
| | 3 | 21.3 mg/mL | 21.0 mg/mL | 21.0 mg/mL | 20.7mg/mL |
| | 6 | 21.1 mg/mL | 20.7 mg/mL | 20.8 mg/mL | 20.6mg/mL |
| | 9 | 21.7mg/mL | 21.0 mg/mL | 21.1 mg/mL | 20.5mg/mL |
| | 12 | 21.6mg/mL | 20.9 mg/mL | 21.1 mg/mL | 20.1mg/mL |
| | 18 | 21.9mg/mL | 21.6 mg/mL | 21.7 mg/mL | NR |
| | 24 | 21.3mg/mL | 20.0 mg/mL | 19.9 mg/mL | NR |
| Insoluble microparticles | 0 | ≥ 10 µm microparticles: 32 microparticles/v ial ≥ 25 µm microparticles: 2 microparticles/v ial | ≥ 10 µm microparticles: 17 microparticles/v ial ≥ 25 µm microparticles: 6 microparticles/v ial | ≥ 10 µm microparticles: 10 microparticles/v ial ≥ 25 µm microparticles: 0 microparticles/v ial | ≥ 10 µm microparticles: 69 microparticles/v ial ≥ 25 µm microparticles: 7 microparticles/v ial |
| | 1 | ≥ 10 µm microparticles: 67 | NA | NA | ≥ 10 µm microparticles: 12 |
| | | microparticles/v ial ≥ 25 µm microparticles: 1 microparticle/vi al | | | microparticles/v ial ≥ 25 µm microparticles: 0 microparticles/v ial |
| | 2 | ≥ 10 µm microparticles: 3 microparticles/v ial ≥ 25 µm microparticles: 0 microparticles/v ial | NA | NA | ≥ 10 µm microparticles: 71 microparticles/v ial ≥ 25 µm microparticles: 3 microparticles/v ial |
| | 3 | ≥ 10 µm microparticles: 14 microparticles/v ial ≥ 25 µm microparticles: 1 microparticle/vi al | ≥ 10 µm microparticles: 3 microparticles/v ial ≥ 25 µm microparticles: 2 microparticles/v ial | ≥ 10 µm microparticles: 4 microparticles/v ial ≥ 25 µm microparticles: 1 microparticle/vi al | ≥ 10 µm microparticles: 55 microparticles/v ial ≥ 25 µm microparticles: 3 microparticles/v ial |
| | 6 | ≥ 10 µm microparticles: 27 microparticles/v ial ≥ 25 µm microparticles: 2 microparticles/v ial | ≥ 10 µm microparticles: 2 microparticles/v ial ≥ 25 µm microparticles: 0 microparticles/v ial | ≥ 10 µm microparticles: 11 microparticles/v ial ≥ 25 µm microparticles: 2 microparticles/v ial | ≥ 10 µm microparticles: 27 microparticles/v ial ≥ 25 µm microparticles: 4 microparticles/v ial |
| | 9 | ≥ 10 µm microparticles: 39 microparticles/v ial ≥ 25 µm | ≥ 10 µm microparticles: 7 microparticles/v ial | ≥ 10 µm microparticles: 4 microparticles/v ial | ≥ 10 µm microparticles: 38 microparticles/v ial |
| | | microparticles: 12 microparticles/v ial | ≥ 25 µm microparticles: 2 microparticles/v ial | ≥ 25 µm microparticles: 1 microparticle/vi al | ≥ 25 µm microparticles: 1 microparticle/vi al |
| | 12 | ≥ 10 µm microparticles: 408 microparticles/v ial ≥ 25 µm microparticles: 28 microparticles/v ial | ≥ 10 µm microparticles: 13 microparticles/v ial ≥ 25 µm microparticles: 5 microparticles/v ial | ≥ 10 µm microparticles: 8 microparticles/v ial ≥ 25 µm microparticles: 2 microparticles/v ial | ≥ 10 µm microparticles: 69 microparticles/v ial ≥ 25 µm microparticles: 7 microparticles/v ial |
| | 18 | ≥ 10 µm microparticles: 71 microparticles/v ial ≥ 25 µm microparticles: 48 microparticles/v ial | ≥ 10 µm microparticles: 41 microparticles/v ial ≥ 25 µm microparticles: 34 microparticles/v ial | ≥ 10 µm microparticles: 12 microparticles/v ial ≥ 25 µm microparticles: 5 microparticles/v ial | NR |
| | 24 | ≥ 10 µm microparticles: 31 microparticles/v ial ≥ 25 µm microparticles: 20 microparticles/v ial | ≥ 10 µm microparticles: 15 microparticles/v ial ≥ 25 µm microparticles: 6 microparticles/v ial | ≥ 10 µm microparticles: 16 microparticles/v ial ≥ 25 µm microparticles: 2 microparticles/v ial | NR |

| | | | | | |
|---|---|---|---|---|---|
| Note: NA in Table represents the non-significant test item at the time point, no detection is performed at the time point, and NR indicates that the time point is not reached. The same applies hereinafter. | | | | | |

### Example 5: Accelerated Stability Study of Formulations

Four batches of stock solutions were selected for formulating the formulations of formula No. 27 as shown in Table 7, then the formulations were stored in transparent vials, and the formulation samples were analyzed after placed under the conditions of 25 ± 2 °C and relative humidity (RH) 60 ± 5% for 0-12 months.

As shown in Table 9, formulations of formula No. 27 have higher stability against protein degradation, and the resulting degradation kinetic parameters measured at 25 ± 2 °C meet the requirements of storage in room temperature environment for up to 12 months.

**Table 9. Accelerated stability data for formulations**

| Test item | Time (mont h) | Result | | | |
|---|---|---|---|---|---|
| | | Batch 1 | Batch 2 | Batch 3 | Batch 4 |
| Appearance | 0 | Colorless and clear liquid with slight opalescence | Colorless and clear liquid with slight opalescence | Colorless and clear liquid with slight opalescence | Colorless and clear liquid with slight opalescence |
| | 1 | Colorless and clear liquid with slight opalescence | Colorless and clear liquid with slight opalescence | Colorless and clear liquid with slight opalescence | Colorless and clear liquid with slight opalescence |
| | 2 | Colorless and clear liquid with slight opalescence | Colorless and clear liquid with slight opalescence | Colorless and clear liquid with slight opalescence | Colorless and clear liquid with slight opalescence |
| | 3 | Colorless and clear liquid with slight opalescence | Colorless and clear liquid with slight opalescence | Colorless and clear liquid with slight opalescence | Colorless and clear liquid with slight opalescence |
| | 6 | Colorless and clear liquid with slight opalescence | Colorless and clear liquid with slight opalescence | Colorless and clear liquid with slight opalescence | Colorless and clear liquid with slight opalescence |
| | 9 | Colorless and clear liquid with slight opalescence | NA | NA | Colorless and clear liquid with slight opalescence |
| | 12 | Colorless and clear liquid with slight opalescence | Colorless and clear liquid with slight opalescence | Colorless and clear liquid with slight opalescence | Colorless and clear liquid with slight opalescence |
| pH | 0 | 5.6 | 5.6 | 5.6 | 5.7 |
| | 1 | 5.6 | 5.6 | 5.6 | 5.7 |
| | 2 | 5.6 | 5.6 | 5.6 | 5.7 |
| | 3 | 5.6 | 5.4 | 5.4 | 5.6 |
| | 6 | 5.6 | 5.6 | 5.5 | 5.6 |
| | 9 | 5.6 | NA | NA | NR |
| | 12 | 5.7 | 5.7 | 5.6 | NR |
| | 0 | 99.7% | 99.7% | 99.7% | 99.7% |
| | 1 | 99.7% | 99.6% | 99.7% | 99.6% |
| SEC-HPLC monomer content (%) | 2 | 99.7% | 99.7% | 99.7% | 99.6% |
| | 3 | 99.7% | 99.6% | 99.6% | 99.5% |
| | 6 | 99.6% | 99.6% | 99.5% | 99.4% |
| | 12 | 99.6% | 99.4% | 99.3% | NR |
| SEC-HPLC polymer content (%) | 0 | 0.3% | 0.3% | 0.3% | 0.3% |
| | 1 | 0.3% | 0.4% | 0.3% | 0.3% |
| | 2 | 0.3% | 0.3% | 0.3% | 0.4% |
| | 3 | 0.3% | 0.3% | 0.3% | 0.4% |
| | 6 | 0.3% | 0.4% | 0.4% | 0.5% |
| | 12 | 0.3% | 0.5% | 0.6% | NR |
| SEC-HPLC fragment content (%) | 0 | 0.0% | 0.0% | 0.0% | 0.0% |
| | 1 | 0.0% | 0.0% | 0.0% | <0.1% |
| | 2 | 0.0% | 0.0% | 0.0% | <0.1% |
| | 3 | 0.0% | 0.0% | 0.0% | 0.1% |
| | 6 | 0.1% | 0.0% | 0.0% | <0.1% |
| | 12 | 0.1% | 0.1% | 0.1% | NR |
| CEX-HPLC main peak content (%) | 0 | 63.7% | 62.6% | 61.7% | 63.1% |
| | 1 | 68.2% | 73.5% | 72.9% | 69.2% |
| | 2 | 70.8% | 71.6% | 71.4% | 69.6% |
| | 3 | 69.2% | 69.0% | 69.1% | 67.3% |
| | 6 | 62.9% | 63.8% | 63.4% | 58.4% |
| | 12 | 50.4% | 50.7% | 50.2% | NR |
| CEX-HPLC acidic peak content (%) | 0 | 10.0% | 13.1% | 12.6% | 12.0% |
| | 1 | 15.2% | 15.6% | 15.2% | 16.0% |
| | 2 | 16.9% | 19.1% | 18.6% | 18.7% |
| | 3 | 19.8% | 21.7% | 21.4% | 21.4% |
| | 6 | 26.9% | 28.4% | 28.2% | 30.5% |
| | 12 | 38.8% | 40.0% | 39.8% | NR |
| CEX-HPLC basic peak content (%) | 0 | 26.3% | 24.3% | 25.7% | 24.9% |
| | 1 | 16.6% | 10.9% | 11.8% | 14.8% |
| | 2 | 12.4% | 9.3% | 10.0% | 11.6% |
| | 3 | 11.0% | 9.3% | 9.5% | 11.3% |
| | 6 | 10.2% | 7.8% | 8.4% | 11.1% |
| | 12 | 10.8% | 9.4% | 10.0% | NR |
| R-CE-SDS content (%) | 0 | 99.9% | 99.8% | 99.7% | 99.6% |
| | 1 | 99.8% | 99.8% | 99.8% | 99.4% |
| | 2 | 99.9% | 99.6% | 99.7% | 99.3% |
| | 3 | 99.6% | 99.7% | 99.6% | 99.4% |
| | 6 | 99.4% | 99.1% | 99.1% | 99.0% |
| | 12 | 98.1% | 98.0% | 97.8% | NR |
| NR-CE-SDS content (%) | 0 | 99.1% | 98.6% | 99.4% | 96.4% |
| | 1 | 98.6% | 97.7% | 98.2% | 97.4% |
| | 2 | 98.5% | 97.4% | 98.3% | 97.3% |
| | 3 | 98.7% | 98.3% | 98.7% | 96.6% |
| | 6 | 98.0% | 97.7% | 97.8% | 92.4% |
| | 12 | 97.0% | 96.2% | 96.5% | NR |
| Biological activity (by ELISA) % | 0 | 107.4% | 101% | 100% | 98% |
| | 1 | NA | 92% | 92% | 100% |
| | 2 | NA | 98% | 97% | 100% |
| | 3 | NA | 97% | 98% | 100% |
| | 6 | 104.0% | 94% | 95% | 92% |
| | 12 | 97.4% | 98% | 94% | NR |
| Protein content (ultraviolet spectrophotomet ry) | 0 | 21.3 mg/mL | 20.9 mg/mL | 20.9 mg/mL | 20.3mg/mL |
| | 1 | 21.5 mg/mL | 20.8 mg/mL | 20.6 mg/mL | 20.5mg/mL |
| | 2 | 21.9 mg/mL | 20.8 mg/mL | 20.7 mg/mL | 20.5mg/mL |
| | 3 | 21.7 mg/mL | 20.9 mg/mL | 20.8 mg/mL | 20.6mg/mL |
| | 6 | 21.9 mg/mL | 20.8 mg/mL | 20.6 mg/mL | 20.7mg/mL |
| | 12 | 21.2 mg/mL | 20.3 mg/mL | 21.0 mg/mL | NR |
| Insoluble microparticles | 0 | ≥ 10 µm microparticles: 32 microparticles/v ial ≥ 25 µm microparticles: 2 microparticles/v ial | ≥ 10 µm microparticles: 17 microparticles/v ial ≥ 25 µm microparticles: 6 microparticles/v ial | ≥ 10 µm microparticles: 10 microparticles/v ial ≥ 25 µm microparticles: 0 microparticles/v ial | ≥ 10 µm microparticles: 69 microparticles/v ial ≥ 25 µm microparticles: 7 microparticles/v ial |
| | 1 | ≥ 10 µm microparticles: 27 microparticles/v ial ≥ 25 µm microparticles: | ≥ 10 µm microparticles: 11 microparticles/v ial ≥ 25 µm microparticles: | ≥ 10 µm microparticles: 8 microparticles/v ial ≥ 25 µm microparticles: | ≥ 10 µm microparticles: 21 microparticles/v ial ≥ 25 µm microparticles: |
| | | 2 microparticles/v ial | 8 microparticles/v ial | 1 microparticle/vi al | 1 microparticle/vi al |
| | 2 | ≥ 10 µm microparticles: 108 microparticles/v ial ≥ 25 µm microparticles: 88 microparticles/v ial | ≥ 10 µm microparticles: 2 microparticles/v ial ≥ 25 µm microparticles: 0 microparticles/v ial | ≥ 10 µm microparticles: 2 microparticles/v ial ≥ 25 µm microparticles: 0 microparticles/v ial | ≥ 10 µm microparticles: 26 microparticles/v ial ≥ 25 µm microparticles: 7 microparticles/v ial |
| | 3 | ≥ 10 µm microparticles: 14 microparticles/v ial ≥ 25 µm microparticles: 0 microparticles/v ial | ≥ 10 µm microparticles: 3 microparticles/v ial ≥ 25 µm microparticles: 1 microparticle/vi al | ≥ 10 µm microparticles: 3 microparticles/v ial ≥ 25 µm microparticles: 0 microparticles/v ial | ≥ 10 µm microparticles: 20 microparticles/v ial ≥ 25 µm microparticles: 0 microparticles/v ial |
| | 6 | ≥ 10 µm microparticles: 109 microparticles/v ial ≥ 25 µm microparticles: 66 microparticles/v ial | ≥ 10 µm microparticles: 11 microparticles/v ial ≥ 25 µm microparticles: 4 microparticles/v ial | ≥ 10 µm microparticles: 12 microparticles/v ial ≥ 25 µm microparticles: 5 microparticles/v ial | ≥ 10 µm microparticles: 25 microparticles/v ial ≥ 25 µm microparticles: 7 microparticles/v ial |
| | 12 | ≥ 10 µm microparticles: 92 microparticles/v ial ≥ 25 µm microparticles: 6 microparticles/v ial | ≥ 10 µm microparticles: 12 microparticles/v ial ≥ 25 µm microparticles: 5 microparticles/v ial | ≥ 10 µm microparticles: 25 microparticles/v ial ≥ 25 µm microparticles: 7 microparticles/v ial | NR |

| | | | | | |
|---|---|---|---|---|---|
| Note: NA in Table represents the non-significant test item at the time point, no detection is performed at the time point, and NR indicates that the time point is not reached. | | | | | |

### Example 6: Detection of the Binding of Humanized Antibodies to hBTLA by ELISA

The binding specificity of the humanized antibodies hu17, hu18 and hu19 to hBTLA was detected by conventional ELISA. 0.5 µg/mL of hBTLA was immobilized on a 96-well plate and incubated at 37 °C for 60 minutes. The solution in wells was then discarded, and the wells were washed 3 times with a washing buffer and blocked for 60 minutes with PBS containing 2% BSA. After the wells were washed 3 times with washing buffer, gradiently-diluted antibody solutions were added (the components other than the antibody in the dilution were the same as those in formula No. 27), incubated at 37 °C for 60 minutes, and washed 3 times with washing buffer before addition of an HRP-labeled mouse anti-human IgG4 secondary antibody diluted at 1:10000. The mixture was incubated at 37 °C for 1 hour, washed three times with washing buffer, and added with 100 µL of TMB substrate solution for color development at room temperature for 30 minutes; then the reaction was stopped with 100 µL of 2 M hydrochloric acid solution and the absorbance was read at 450 nm.

The EC₅₀ values are shown in Table 10 below. The results show that the humanized antibodies hu17, hu18 and hu19 can specifically bind to hBTLA.

**Table 10. Data on the specific binding of humanized antibodies hu17, hu18 and hu19 to hBTLA**

| Humanized antibody | hu17 | hu18 | hu19 |
|---|---|---|---|
| EC₅₀(ng/mL) | 6.3 | 5.4 | 6.6 |

### Example 7. Detection of the Binding of Humanized Antibodies to hBTLA on 293F Cells by FACS

The humanized anti-BTLA antibodies hu17, hu18 and hu19 were determined for their binding ability to hBTLA expressed on cells using cell-based flow cytometry (FACS) assay. 293F cells expressing hBTLA were centrifuged and resuspended in an FACS buffer after digestion, and then added into a 96-well round-bottom plate at a density of 2.5 × 10⁴/50 µL. Then the cells were mixed with 50 µL of antibody diluent at different concentrations (initial concentration: 10 µg/mL, 3-fold titration, the components other than the antibody in the diluent were the same as those in formula No. 27), and incubated at room temperature for 30 minutes. After being washed with an FACS buffer twice, the cells were added with 100 µL of goat anti-human IgG-PE antibody, incubated for 30 minutes away from light, and detected by FACS after two washes with an FACS buffer. The washed cells were resuspended in 4 °C buffer containing propidium iodide (PI) and 0.02% sodium azide that prevents receptor internalization and analyzed by flow cytometry. Viable cells were gated on the basis of PI positive cells being excluded from the FSC/SSC gate and their geometric mean fluorescence intensity was measured. The data were analyzed using a sigmoidal dose-response model within Prism^{™} software.

The EC₅₀ values for each antibody are shown in Table 11 below. The results show that the humanized antibodies hu17, hu18 and hu19 effectively bind to hBTLA on 293F cells.

**Table 11**

| | hu17 | hu18 | hu19 |
|---|---|---|---|
| EC₅₀ (ng/mL) | 96.74 | 89.34 | 95.06 |

### Example 8: Detection of Humanized Antibodies' Blocking of the Binding of BTLA to HVEM by FACS

The humanized antibodies hu17, hu18 and hu19 were determined for their ability to block the binding of hBTLA to hHVEM expressed on 293F cells using cell-based flow cytometry (FACS) assay. hHVEM-293F stable cells were centrifuged and resuspended in an FACS buffer after digestion, and then added into a 96-well round-bottom plate at a density of 2.5 × 10⁴ cells/50 µL. Then the cells were mixed with previously biotin-labeled hBTLA (1 µg/mL) protein, incubated at 4 °C for 15 minutes, followed by mixing with 50 µL of humanized antibody diluent (initial concentration: 5 µg/mL, 3-fold titration, the components other than the antibody in the diluent were the same as those in formula No. 27) at different concentrations, and incubating at room temperature for 30 minutes. After being washed with an FACS buffer twice, the cells were added with 100 µL of goat anti-human IgG-PE antibody, incubated for 30 minutes away from light, and detected by FACS after two washes with an FACS buffer. The washed cells were resuspended in 4 °C buffer containing propidium iodide (PI) and 0.02% sodium azide that prevents receptor internalization and analyzed by flow cytometry. Viable cells were gated on the basis of PI positive cells being excluded from the FSC/SSC gate and their geometric mean fluorescence intensity was measured. The data were analyzed using a sigmoidal dose-response model within Prism^{™} software.

The IC₅₀ values for each antibody are shown in Table 12 below. The results show that the humanized antibodies hu17, hu18 and hu19 can effectively block the binding of BTLA to HVEM on the cell surface.

**Table 12**

| | hu17 | hu18 | hu19 |
|---|---|---|---|
| IC₅₀ (ng/mL) | 142.7 | 172.2 | 161.5 |

### Example 9. Promotion of T cell Activation by Humanized Anti-BTLA Antibodies

CHO cells stably expressing hPD-L1/hHVEM were spread on a 96-well plate at 5 × 10⁴ cells/well, and incubated overnight at 37 °C, 7% CO₂. After the cell supernatant was removed, each well was added with 40 µL of humanized anti-BTLA antibody diluent (initial concentration: 60 µg/mL, 3-fold gradient dilution, the components other than the antibody in the diluent were the same as those in formula No. 27), and 40 µL of Jurkat report cells capable of continuously expressing hPD-1/hBTLA/NFAT-luciferase, with a total cell number being 1 × 10⁵ cells. The mixture was incubated at 37 °C for 6 hours, 7% CO₂, and added with luciferase reagent. The luminescence values were detected by a microplate reader.

The EC₅₀ values for each antibody are shown in Table 13 below. The results show that the humanized anti-BTLA antibodies hu17, hu18 and hu19 can effectively promote T cell activation.

**Table 13**

| | hu17 | hu18 | hu19 |
|---|---|---|---|
| EC₅₀ (ng/mL) | 256.7 | 277.3 | 290.4 |

### Example 10. Affinity of Humanized Anti-BTLA Antibodies for hBTLA

The assay was carried out using a Biacore T200 instrument (GE Healthcare). Series S CM5 chip was loaded on the instrument and the system buffer used was HBS-EP+ (10 mM HEPES, pH 7.4, 150 mM NaCl, 3 mM EDTA, 0.05% surfactant P20). The BTLA-Fc antigens were coupled in a chip detection channel: injecting a mixed solution of 400 mM EDC and 100 mM NHS at 10 µL/min for 420 s to activate the surface of the chip, diluting the BTLA-Fc antigens in a 10 mM sodium acetate/acetic acid (pH 5.5) buffer to a final concentration of 20 µg/mL and injecting at 10 µL/min for coupling, and injecting a 1 M ethanolamine-hydrochloric acid solution (pH 8.5) at 10 µL/min for 420 s for blocking.

Antibodies were diluted in a 2-fold gradient with Biacore system buffer for a total of 6 concentration points (the components other than the antibody in the diluent were the same as those in formula No. 27). Concentration gradients were 24 nM, 12 nM, 6 nM, 3 nM, 1.5 nM and 0.75 nM, with 24 nM being a replicate. Data were analyzed using Biacore T200 Evaluation Software (version no. 3.0, GE Healthcare). Data were fitted using a model of 1:1 Binding. The kinetic constants of the binding of the antibodies to the antigens, namely, the association rate constant ka (1/Ms), dissociation rate constant kd (1/s) and the affinity constant KD (M), were obtained by fitting, and the results are shown in Table 14.

**Table 14. Affinity data for humanized antibodies**

| | ka(1/Ms) | kd(1/s) | KD(M) |
|---|---|---|---|
| hu17 | 6.24E+05 | 1.16E-04 | 1.86E-10 |
| hu18 | 6.39E+05 | 8.67E-05 | 1.36E-10 |

### Example 11. Characterization of Binding Kinetics of Humanized Antibodies to BTLAs of Different Species

To detect cross-reactivity between chimeric antibodies and cynomolgus and murine BTLAs, Fortebio assay was used. Briefly, human BTLA, cynomolgus BTLA or murine BTLA was coupled to an activated CM5 biosensor chip to achieve approximately 100-200 response units (RUs), and then the unreacted groups were blocked with IM ethanolamine. Humanized antibody samples (antibody in the samples was hu17, and other components therein were the same as those in formula No. 27) at increasing concentrations from 0.12 nM to 90 nM were injected at 30 times/min into SPR running buffer and binding responses at BTLAs of different species were calculated by subtracting RU from the blank flow cell.

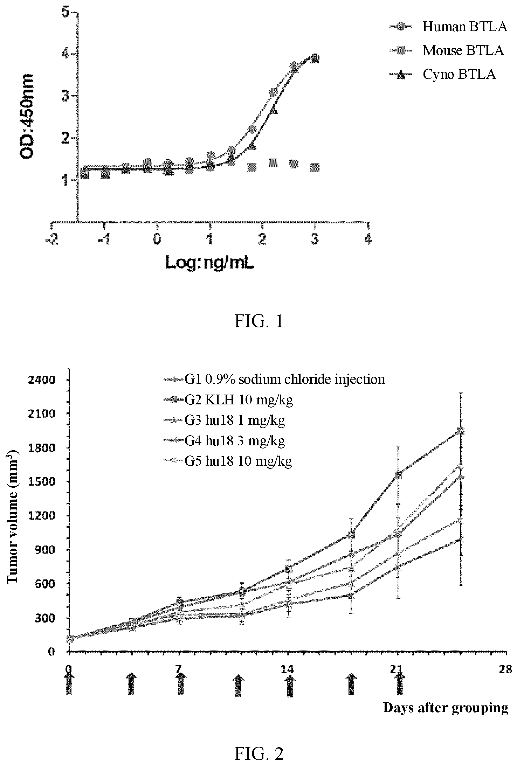

The results are shown in FIG. 1. Comparative results of hu17's binding to BTLAs of different species show that: hu17 not only has high affinity for human BTLA, but also has similar affinity for cynomolgus monkey BTLA, and it barely binds to murine BTLA.

### Example 12: Inhibition of Tumor Growth in Mice by Humanized Antibodies (Formula No. 27)

The MC38-hHVME cell bank was created on MC38 cells (ATCC) by electroporating Hxp-hHVEM plasmids, the cells were subcloned by limiting dilution method, and the monoclones were screened by flow cytometry to acquire MC38-HVEM cells. The MC38-HVEM cells were inoculated into the right subcutaneous tissue of the humanized female B-HBTLA mice at 1 × 10⁶ cells/0.1 mL, and when tumors grew to approximately 118 mm³, the mice were randomly grouped, by tumor volume, into 5 groups (8 mice per group), namely G1 0.9% sodium chloride injection solvent control group, G2 KLH (anti-keyhole limpet hemocyanin antibody ) (10 mg/kg) negative control group, G3 hu18 (1 mg/kg) group, G4 hu18 (3 mg/kg) group, and G5 hu18 (10 mg/kg) group. All groups were subjected to administration by intraperitoneal injection twice a week for 7 consecutive times, and the experiment ended 4 days after the last administration. Tumor volume and body weight of mice were measured and recorded twice a week. At the end of the experiment, animals were euthanized, tumors were taken, weighed and photographed, and the relative tumor inhibition (TGI%, TGI% = (1-(Tᵢ-T₀)/(Vᵢ-V₀))×100%, wherein Tᵢ is the tumor volume at the end of administration of the treatment group, T₀ is the tumor volume at the beginning of administration of the treatment group, Vᵢ is the tumor volume at the end of administration of the blank group, and V₀ is the tumor volume at the beginning of administration of the blank group) was calculated.

The effect of each test sample on the tumor volume of MC38-hHVEM cell transplanted B-hBTLA mice is shown in Table 15 and FIG. 2. 21 days after the first administration, the KLH (10 mg/kg) negative control group had an mean tumor volume of 1560 ± 256 mm³, and the other dosing groups had mean tumor volumes of 1073 ± 224 mm³, 747 ± 268 mm³ and 868 ± 211 mm³. Compared with the KLH negative control group, the other dosing groups had the TGI% of 33.7%, 56.4% and 48.0%, and P values of 0.175, 0.046 and 0.056, indicating that the test drug hu18 has a certain inhibitory effect on tumor growth.

**Table 15. Effect of hu18 on the tumor volume of MC38-hHVEM cell-transplanted B-hBTLA mice**

| Groups | Test drug | Tumor volume (mm³)^{a} | | | *P*^{b} |
|---|---|---|---|---|---|
| | | Before administration | 21 days after the first administration | TGI (%) | |
| G1 | 0.9% sodium chloride injection | 118±2 | 1029±155 | - | - |
| G2 | KLH (10 mg/kg) | 118±2 | 1560±256 | - | - |
| G3 | Antibody hu18 (1 mg/kg) | 118±3 | 1073±224 | 33.7 | 0.175 |
| G4 | Antibody hu18 (3 mg/kg) | 118±2 | 747±268 | 56.4 | 0.046 |
| G5 | Antibody hu18 (10 mg/kg) | 118±3 | 868±211 | 48.0 | 0.056 |

| | | | | | |
|---|---|---|---|---|---|
| Note: a: mean ± standard error; b: the statistical comparison of the tumor volume of the dosing groups with tumor volumes of the KLH negative control group at 21 days after the administration, t-test. | | | | | |

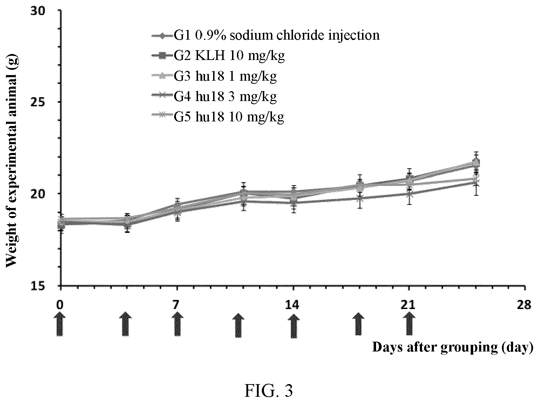

The results of the effect of each test sample on the body weight of MC38-hHVEM cell-transplanted B-hBTLA mice are shown in Table 16 and FIG. 3. All experimental animals were well active and fed during administration, and the body weight of animals in each dosing group increased to some extent. No mortality of the experimental animals occurred during the experiment. Compared with the body weight of mice in the KLH negative control group, the body weight of the mice in each dosing group had no significant change (P>0.05) 21 days after the administration, indicating that the experimental animals have good tolerance to the samples.

**Table 16. Effect of test samples on the body weight of MC38-hHVEM cell-transplanted B-hBTLA mice**

| Groups | Test drug | Body weight (g) ^{a} | | | Body weight change (g) 21 days after administration |
|---|---|---|---|---|---|
| | | Before administration | 21 days after the first administration | *P*^{b} | |
| G1 | 0.9% sodium chloride injection | 18.4±0.4 | 20.7±0.4 | - | +2.3 |
| G2 | KLH (10 mg/kg) | 18.3±0.5 | 20.8±0.6 | - | +2.5 |
| G3 | hu18 (1 mg/kg) | 18.5±0.4 | 20.8±0.4 | 0.943 | +2.3 |
| G4 | hu18 (3 mg/kg) | 18.5±0.4 | 20.0±0.6 | 0.341 | +1.5 |
| G5 | hu18 (10 mg/kg) | 18.6±0.3 | 20.5±0.2 | 0.623 | +1.9 |

| | | | | | |
|---|---|---|---|---|---|
| Note: a: mean ± standard error; b: body weights of the dosing groups compared statistically with that of the KLH negative control group 21 days after administration, t-test. | | | | | |

### Example 13: Humanized Antibody hu18 (Formula No. 27) without ADCC effector function

ADCC is initiated when an antibody binds to a cell surface target protein and then links to an Fc γ receptor (FcγR) expressed on effector cells. It is clearly documented that human IgG1 has significantly higher binding affinity for FcγR than IgG4, particularly for FcγR-I and FcγR-IIIA, which correlates with the strength of IgG1 to activate ADCC. In conjunction with ADCC, CDC is activated when antibodies cross-link the cell surface target and C1q protein, followed by a cascade reaction of complement complex formation and target cell lysis. As a proxy for ADCC and CDC, the detection of antibody binding to FcγR and C1q may serve as basic indicators of ADCC and CDC. Thus, in the present invention, the kinetic binding affinity of monoclonal antibodies for major FcγRs was evaluated using biacore T200 (GE).

Anti-His antibody (GE) was immobilized on the sensor wafer. Various Fc receptors, including recombinant human FcγRIIIA (CD16a) V176, recombinant human FcyRIIA (CD32a) R167, recombinant human FcγRI (CD64) and recombinant human FcRn were captured, then a series of diluted recombinant human anti-BTLA antibodies (i.e., hu18) were injected, and the binding properties of the interactions were assayed and analyzed. hu18 is an IgG4 subtype, and hu18-IgG1 is an IgG1 subtype that shares the same Fab as hu18 and serves as a positive control.

As shown in Table 17, the binding of the recombinant human anti-BTLA antibody of the IgG4 subtype to Fc receptors was relatively weak compared to that of the control antibody of the IgG1 subtype, and the interaction of the hu18 antibody with FcγRIIIA (CD16a) V176 was 400-fold weaker compared to that of the control antibody of the IgG1 subtype. This suggests that hu18 has weak or no ADCC effect. As shown in Table 17, the hu18 antibody did not bind to C1q, while the hu18-IgG1 antibody was able to bind to C1q.

**Table 17. Binding affinity of interaction between anti-BTLA antibodies and Fc receptors**

| Fc receptor | Antibody | Ka (1/Ms) | Kd (1/s) | KD (M) |
|---|---|---|---|---|
| FcγRIIIA (CD16a) V176 | hu18-IgG1 | 6.15E+04 | 8.85E-03 | 1.44E-07 |
| | hu18 | N/A | N/A | 6.02E-05 |
| FcγRIIA (CD32a) R167 | hu18-IgG1 | N/A | N/A | 5.90E-06 |
| | hu18 | N/A | N/A | 4.06E-05 |
| FcγRI (CD64) | hu18-IgG1 | 2.99E+05 | 1.20E-03 | 4.00E-09 |
| | hu18 | 2.10E+05 | 3.87E-03 | 1.84E-08 |
| FcRn | hu18-IgG1 | 1.69E+06 | 3.88E-02 | 2.29E-08 |
| | hu18 | 5.12E+05 | 7.08E-02 | 1.38E-07 |

**Table 18. Binding affinity of interaction between anti-BTLA antibodies and human C1q**

| Protein | Antibody | Conclusion |
|---|---|---|
| C1q | hu18-IgG1 | Specific binding |
| | hu18 | Non-specific binding |

## Claims

1. A pharmaceutical composition, comprising:
(1) a buffer; and
(2) an anti-BTLA antibody or an antigen-binding fragment thereof, wherein the anti-BTLA antibody or the antigen-binding fragment thereof has an HCDR1, an HCDR2 and an HCDR3 having amino acid sequences set forth in SEQ ID NO: 1, SEQ ID NO: 2 and SEQ ID NO: 3, respectively, and an LCDR1, an LCDR2 and an LCDR3 having amino acid sequences set forth in SEQ ID NO: 4, SEQ ID NO: 5 and SEQ ID NO: 6, respectively.

2. The pharmaceutical composition according to claim 1, wherein the buffer is selected from an acetic acid buffer, a citric acid buffer and a histidine buffer; preferably, the buffer is a histidine buffer; and more preferably, the buffer is a histidine-hydrochloride buffer, more preferably a histidine-histidine hydrochloride buffer.

3. The pharmaceutical composition according to claim 2, wherein the buffer has a concentration of about 10 mM to 30 mM; preferably, the buffer has a pH of about 5.0-6.5, preferably about 5.0-6.0.

4. The pharmaceutical composition according to any one of claims 1-3, wherein the pharmaceutical composition further comprises a stabilizer selected from one or more of sodium chloride, mannitol, sorbitol, sucrose, maltose, xylitol and trehalose; preferably, the stabilizer is a combination of trehalose and sodium chloride.

5. The pharmaceutical composition according to claim 4, wherein the stabilizer is sodium chloride at a concentration of about 50-200 mM; or the stabilizer is mannitol at a concentration of about 100-300 mM; or the stabilizer is sorbitol at a concentration of about 100-300 mM; or the stabilizer is sucrose at a concentration of about 100-300 mM; or the stabilizer is trehalose at a concentration of about 100-300 mM; or the stabilizer is a combination of about 30-100 mM sodium chloride and about 50-200 mM mannitol; or the stabilizer is a combination of about 30-100 mM sodium chloride and about 50-200 mM sorbitol; or the stabilizer is a combination of about 30-100 mM sodium chloride and about 50-200 mM sucrose; or the stabilizer is a combination of about 30-100 mM sodium chloride and about 50-200 mM trehalose; preferably, the stabilizer is the combination of about 30-100 mM sodium chloride and about 50-200 mM trehalose.

6. The pharmaceutical composition according to any one of claims 1-5, wherein the pharmaceutical composition further comprises a surfactant selected from polysorbate 80, polysorbate 20 and poloxamer 188, preferably, the surfactant is polysorbate 80.

7. The pharmaceutical composition according to claim 6, wherein the surfactant has a concentration of about 0.01%-0.1%; preferably, the surfactant has a concentration of about 0.01%-0.05%.

8. The pharmaceutical composition according to any one of claims 1-7, wherein the anti-BTLA antibody has a heavy chain variable region having an amino acid sequence set forth in SEQ ID NO: 7 and a light chain variable region having an amino acid sequence set forth in SEQ ID NO: 8; or has a heavy chain variable region having an amino acid sequence set forth in SEQ ID NO: 7 and a light chain variable region having an amino acid sequence set forth in SEQ ID NO: 9; or has a heavy chain variable region having an amino acid sequence set forth in SEQ ID NO: 7 and a light chain variable region having an amino acid sequence set forth in SEQ ID NO: 10.

9. The pharmaceutical composition according to any one of claims 1-7, wherein the anti-BTLA antibody has a heavy chain amino acid sequence set forth in SEQ ID NO: 11 and a light chain amino acid sequence set forth in SEQ ID NO: 12.

10. The pharmaceutical composition according to any one of claims 1-9, wherein the anti-BTLA antibody or the antigen-binding fragment thereof has a concentration of about 5-100 mg/mL, preferably about 10-50 mg/mL, and more preferably 15-25 mg/mL.

11. The pharmaceutical composition according to any one of claims 1-9, comprising or consisting of components as shown in any one of (1) to (13) below:
(1) (a) about 10 mg/mL to 50 mg/mL of the anti-BTLA antibody or the antigen-binding fragment thereof; (b) about 10-30 mM histidine buffer at a pH of about 5.0-6.0; (c) a combination of about 30 mM to about 100 mM sodium chloride and about 50-200 mM trehalose; and (d) about 0.01%-0.05% of polysorbate 80;
(2) (a) about 10 mg/mL to 50 mg/mL of the anti-BTLA antibody or the antigen-binding fragment thereof; (b) about 10-30 mM histidine buffer at a pH of about 5.0-6.0; (c) a combination of about 30 mM to about 100 mM sodium chloride and about 50-200 mM mannitol; and (d) about 0.01%-0.05% of polysorbate 80;
(3) (a) about 10 mg/mL to 50 mg/mL of the anti-BTLA antibody or the antigen-binding fragment thereof; (b) about 10-30 mM histidine buffer at a pH of about 5.0-6.0; (c) a combination of about 30 mM to about 100 mM sodium chloride and about 50-200 mM sucrose; and (d) about 0.01%-0.05% of polysorbate 80;
(4) (a) about 10 mg/mL to 50 mg/mL of the anti-BTLA antibody or the antigen-binding fragment thereof; (b) about 10-30 mM histidine buffer at a pH of about 5.0-6.0; (c) a combination of about 30 mM to about 100 mM sodium chloride and about 50-200 mM sorbitol; and (d) about 0.01%-0.05% of polysorbate 80;
(5) (a) about 10 mg/mL to 50 mg/mL of the anti-BTLA antibody or the antigen-binding fragment thereof; (b) about 10-30 mM histidine buffer at a pH of about 5.0-6.0; (c) about 100 mM to about 300 mM trehalose; and (d) about 0.01%-0.05% of polysorbate 80;
(6) (a) about 10 mg/mL to 50 mg/mL of the anti-BTLA antibody or the antigen-binding fragment thereof; (b) about 10-30 mM citric acid buffer at a pH of about 5.5-6.5; (c) about 100 mM to about 300 mM trehalose; and (d) about 0.01%-0.05% of polysorbate 80;
(7) (a) about 10 mg/mL to 50 mg/mL of the anti-BTLA antibody or the antigen-binding fragment thereof; (b) about 10-30 mM citric acid buffer at a pH of about 5.5-6.5; (c) a combination of about 30 mM to about 100 mM sodium chloride and about 50-200 mM mannitol; and (d) about 0.01%-0.05% of polysorbate 80;
(8) (a) about 10 mg/mL to 50 mg/mL of the anti-BTLA antibody or the antigen-binding fragment thereof; (b) about 10-30 mM acetic acid buffer at a pH of about 5.0-6.0; (c) a combination of about 30 mM to about 100 mM sodium chloride and about 50-200 mM mannitol; and (d) about 0.01%-0.05% of polysorbate 80;
(9) (a) about 20 mg/mL of the anti-BTLA antibody or the antigen-binding fragment thereof; (b) about 20 mM histidine buffer at a pH of about 6.0; (c) a combination of about 54 mM sodium chloride and about 132 mM trehalose; and (d) about 0.02% of polysorbate 80;
(10) (a) about 20 mg/mL of the anti-BTLA antibody or the antigen-binding fragment thereof; (b) about 20 mM histidine buffer at a pH of about 5.5; (c) about 220 mM trehalose; and (d) about 0.02% of polysorbate 80;
(11) (a) about 20 mg/mL of the anti-BTLA antibody or the antigen-binding fragment thereof; (b) about 20 mM histidine buffer at a pH of about 5.5; (c) a combination of about 50 mM sodium chloride and about 140 mM mannitol; and (d) about 0.02% of polysorbate 80;
(12) (a) about 20 mg/mL of the anti-BTLA antibody or the antigen-binding fragment thereof; (b) about 20 mM histidine buffer at a pH of about 5.5; (c) a combination of about 50 mM sodium chloride and about 140 mM trehalose; and (d) about 0.02% of polysorbate 80; and
(13) (a) about 20 mg/mL of the anti-BTLA antibody or the antigen-binding fragment thereof; (b) about 20 mM histidine buffer at a pH of about 5.0-6.0; (c) a combination of about 50 mM sodium chloride and about 140 mM trehalose; and (d) about 0.02% of polysorbate 80.

12. The pharmaceutical composition according to claim 1, wherein the pharmaceutical composition comprises or consists of: a histidine-histidine hydrochloride buffer at a pH of 5.5 ± 0.3 and with a concentration of 20 ± 5 mM, 40-80 mM sodium chloride, 120-150 mM trehalose, 0.02-0.04% (w/v) of polysorbate 80, and 15-25 mg/mL of the anti-BTLA antibody or the antigen-binding fragment thereof; wherein the anti-BTLA antibody has a heavy chain amino acid sequence set forth in SEQ ID NO: 11 and a light chain amino acid sequence set forth in SEQ ID NO: 12.

13. Use of the pharmaceutical composition according to any one of claims 1-12 in the preparation of a medicament for treating or preventing BTLA-mediated diseases; preferably, the diseases are tumors, infectious diseases or autoimmune diseases; preferably, the tumors include melanoma, breast cancer, renal cancer, prostate cancer, colon cancer, lung cancer, pancreatic cancer, bone cancer, skin cancer, head or neck cancer, uterine cancer, ovarian cancer, rectal cancer, anal cancer, stomach cancer, testicular cancer, small intestine cancer, cervical cancer, vaginal cancer, Hodgkin's disease, non-Hodgkin's lymphoma, esophageal cancer, cancer of the endocrine system, thyroid cancer, carcinoma of adrenal gland, soft tissue cancer, urethral cancer, chronic or acute leukemia, chronic myeloid leukemia, acute lymphoblastic leukemia, chronic lymphocytic leukemia, childhood solid tumor, lymphocytic lymphoma, bladder cancer, kidney or ureteral cancer, renal pelvis cancer, neoplasms of the central nervous system, primary central nervous system lymphoma, tumor angiogenesis, spinal axis tumor, brain stem glioma, pituitary adenoma, Kaposi's sarcoma, epidermoid carcinoma, squamous cell carcinoma, T-cell lymphoma and environmentally induced cancers; the autoimmune diseases include organ-specific autoimmune diseases and systemic autoimmune diseases, wherein the organ-specific autoimmune diseases include chronic lymphocytic thyroiditis, hyperthyroidism, insulin-dependent diabetes mellitus, myasthenia gravis, ulcerative colitis, pernicious anemia with chronic atrophic gastritis, goodpasture's syndrome, pemphigus vulgaris, pemphigoid, primary biliary cirrhosis, multiple sclerosis and acute idiopathic polyneuritis; and the systemic autoimmune diseases include systemic lupus erythematosus, rheumatoid arthritis, systemic vasculitis, scleroderma, pemphigus, dermatomyositis, mixed connective tissue disease, autoimmune hemolytic anemia and ulcerative colitis.
